# EUROPEAN PATENT APPLICATION

(11) **EP 4 520 367 A1**
(43) Date of publication of application: **12.03.2025**
(21) Application number: 24154456.8
(22) Date of filing: 29.01.2024
(51) Int. Cl.: A61M 5/315, A61M 5/24

(54) **MEDICAMENT DELIVERY MECHANISM**

(71) Applicant: medmix Switzerland AG, 9469 Haag (Rheintal) (CH)
(72) Inventor: KEITEL, Joachim, 73728 Esslingen (DE); BECHTOLD, Herbert, 78588 Denkingen (DE)
(74) Representative: Manitz Finsterwald Patent- und Rechtsanwaltspartnerschaft mbB

(57) **Abstract**

A medicament delivery mechanism for dispensing a medicament from a medicament container comprises a body front part, a body rear part, a dose setting element for setting at least one dose, a dose setting sleeve rotationally fixed to the dose setting element during dose setting, a piston rod extending from the body front part for advancing a plunger within the medicament container to deliver the medicament, a nut, wherein the nut forms a threaded connection with the piston rod, a drive sleeve, wherein the body front part forms a container connector to connect the medicament container to the body front part, wherein the body front part is at least partly disposed within the body rear part, wherein the body front part forms a linear guide for the drive sleeve that rotationally fixes the drive sleeve to the body front part, wherein the body front part and the nut are rotatable to each other during dose setting, wherein the body front part comprises at least one dose stop, wherein the dose stop is configured to hold the nut in a predefined relative rotational position to the body front part, the relative rotational position corresponding to the at least one settable dose, and wherein the body rear part forms an outer housing defining an outer surface of the medicament delivery mechanism.

## Description

### FIELD

The present disclosure relates to medicament delivery mechanisms for dispensing a medicament from a medicament container and to medicament delivery devices comprising the medicament delivery mechanisms.

### BACKGROUND

Medicament delivery mechanisms for dispensing a medicament from a medicament container are configured to deliver at least one predetermined dose of medicament to a patient. Usually, such medicament delivery mechanisms allow to first set the dose to be delivered in a dose setting state of the medicament delivery mechanism and to then deliver a set dose in a dose delivery state of the medicament delivery mechanism.

For dose setting, medicament delivery mechanisms usually comprise a dose setting element that is actuated by a user to set the dose. For example, the dose setting element may be configured to be rotated by the user to set the dose. The medicament delivery mechanism thereby may be configured to allow only a single predetermined dose to be set or several varying predetermined doses. Furthermore, medicament delivery mechanisms usually comprise an actuation element, such as a push button, that is configured to be actuated by the user to deliver a set dose.

To avoid delivery of the medicament during dose setting and to allow the delivery of the medicament during dose delivery, the medicament delivery mechanisms usually comprise one or more members that are configured to move with respect to at least one other member during dose setting and that are restricted from moving with respect to the at least one other member during dose delivery or vice versa. This allows to transfer a force to a delivery member, such as a piston rod, only during dose delivery but not during dose setting.

One such medicament delivery mechanism is exemplarily described in document US20060258988A1. The medicament delivery mechanism thereby comprises a piston rod and a threaded part that is threadedly engaged with the piston rod. During dose setting, the threaded part is configured to rotate with respect to the piston rod and thereby to advance in an axial direction with respect to the piston rod to set a dose. During dose delivery, the threaded part is configured to remain rotationally stationary with respect to the piston rod. Axial movement of the threaded part then pushes the piston rod proximally to deliver a set dose. A rotation of the threaded part is thereby prevented by a flexible detent member that engages with axial splines provided at a housing of the device.

There is a need to simplify production of such a medicament delivery mechanism without sacrificing precision of the medicament delivery mechanism.

In a further aspect, medicament delivery mechanisms usually comprise a maximum dose stop that blocks further movement of the dose setting element by the user during dose setting when a maximum settable dose is reached. Sometimes, a user moves, e.g. turns, the dose setting element with more force than usual and thereby moves, e.g. turns, the dose setting element past a maximum settable dose position. Moving the dose setting element past the maximum settable dose position usually renders the medicament delivery mechanism unusable.

Accordingly, there is a need to improve the reliability of a medicament delivery mechanism in a way that it is less likely for the user to move a dose setting element past the maximum dose stop position.

### SUMMARY

The present disclosure provides a medicament delivery mechanism according to claim 1. Embodiments are given in the dependent claims, the description, and the drawings.

In a first aspect, the present disclosure is directed at a medicament delivery mechanism for dispensing a medicament from a medicament container. The medicament delivery mechanism comprises a body front part, a body rear part, a dose setting element for setting at least one dose, a dose setting sleeve rotationally fixed to the dose setting element during dose setting, a piston rod extending from the body front part for advancing a plunger within the medicament container to deliver the medicament, a nut, wherein the nut forms a threaded connection with the piston rod, and a drive sleeve. The body front part forms a container connector to - directly or indirectly - connect the medicament container to the body front part. According to this application, an indirect connection of two parts exists if the two parts are connected to each other via at least a third part. The body front part is at least partly disposed within the body rear part. In other words, the body rear part surrounds a space in which the body front part extends. The body front part forms a linear guide for the drive sleeve that rotationally fixes the drive sleeve to the body front part. Therefore, the drive sleeve can move linearly relative to the body front part, but is blocked from rotating relative to the body front part. The body front part and the nut are rotatable to each other during dose setting. The body front part comprises at least one dose stop, wherein the dose stop is configured to hold the nut in a predefined relative rotational position to the body front part, the relative rotational position corresponding to the at least one settable dose. The body rear part forms an outer housing defining an outer surface of the medicament delivery mechanism.

According to an embodiment, the body front part and the body rear part form separate members, i.e. separately manufactured parts, that are fixedly connected to each other, e.g. by a non-releasable connection and/or a snap-fit connection, gluing, welding or the like. This inter alia allows simpler manufacturing of the medicament delivery mechanism.

According to an embodiment, the body front part is formed as a single integral piece and/or a one-pieced part. For example, the body front part may be made from a plastic material, such as a thermoplastic material. The body front part may be fabricated as a molded part, such as an injection molded part.

According to an embodiment, the body rear part is formed as a single integral piece and/or a one-pieced part. For example, the body rear part may be made from a plastic material, such as a thermoplastic material. The body rear part may be fabricated as a molded part, such as an injection molded part.

According to an embodiment, the dose setting element is configured to set one of a plurality of settable doses, and the body front part comprises a plurality of dose stops, each of the plurality of dose stops is configured to hold the nut in a predefined relative rotational position to the body front part, each relative rotational position corresponding to one of the plurality of settable doses.

According to an embodiment, the at least one dose stop is located on a circumferential surface of the body front part.

If the dose setting element is configured to set one of a plurality of settable doses, and the body front part comprises a plurality of dose stops, the plurality of dose stops can be evenly distributed along the circumferential surface of the body front part.

According to an embodiment, the at least one dose stop is located on an inner surface of the body front part, e.g. an inner circumferential surface of the body front part.

According to an embodiment, the at least one dose stop is configured to directly engage with the nut to hold the nut in a predefined relative rotational position to the body front part. For example, the nut may be configured to engage with the dose stop via a flexible portion of the nut, such as a flexible and/or radially extending arm.

According to an embodiment, the at least one dose stop forms part of a latching mechanism acting between the body front part and the nut. For example, the latching mechanism is configured to allow rotation of the nut relative to the body front part when a rotational force acting on the nut is larger than a predetermined threshold, i.e. a certain force, but blocks rotation of the nut relative to the body front part when a rotational force acting on the nut is smaller than said predetermined threshold, e.g. that certain force. In other words, the user needs to apply at least a minimum rotational force to the dose setting element to make the nut turn relative to the body front part.

According to an embodiment, the at least one dose stop is configured as a longitudinal ridge, i.e. a ridge with a main direction of extension along the longitudinal axis of the device. In other words, a longitudinal ridge is a ridge that runs parallel to the longitudinal axis of the device.

The dose stop may be configured and/or act as a linear guide for the drive sleeve. With a plurality of dose stops, a multitude of the dose stops, such as two, four, six or eight dose stops, may be configured and/or act as a linear guide for the drive sleeve.

According to an embodiment, the body front part forms a linear guide for the piston rod that rotationally fixes the piston rod to the body front part. For example, the body front part forms a non-round, e.g. oval, opening and the piston rod forms a corresponding non-round, e.g. oval, outer shape.

According to an embodiment, the body rear part forms a threaded connection with the dose setting sleeve.

According to an embodiment, the container connector is a snap-fit connector, such as a non-releasable snap-fit connector.

According to an embodiment, the body front part and the body rear part form corresponding and mutual engaging body connectors to connect the body front part and the body rear part. The body connectors may be configured as snap-fit connectors.

According to an embodiment, the body front part forms an outer surface of the medicament delivery mechanism. The outer surface of the medicament delivery mechanism may be an outer surface of a medicament delivery device. The outer surface can be located proximally from the outer surface formed by the rear body part.

According to an embodiment, the body front part forms a linear guide for the nut. According to an embodiment, said linear guide for the nut is configured to additionally be the linear guide for the drive sleeve. This provides for a compact design of the mechanism.

The linear guide may be formed by the dose stop. With a plurality of dose stops, a plurality of the dose stops, such as each dose stop, may form the linear guide for the nut.

The body front part, such as the one or more dose stops, may act as a linear guide for the nut during dispensing, such as only during dispensing. During dose setting, body front part, such as the one or more dose stops, may not act as a linear guide for the nut but as a rotational stop of a rotational movement of the nut. For example, the medicament delivery mechanism may be configured to transfer a force exerted by a user during dose setting as a torque to the nut which forces the nut to rotate with respect to the body front part. For example, the nut may be rotationally locked to the dose setting element and/or the dose setting sleeve during dose setting. The medicament delivery mechanism may be configured to transfer a force exerted by a user to the nut as a force acting linearly along a longitudinal axis of the device during dispensing. The force, may be, for example transferred by the drive sleeve pushing on the nut. During dispensing, the nut may be rotationally decoupled from the dose setting element and/or the dose setting sleeve.

According to an embodiment, the linear guide for the drive sleeve formed by the body front part comprises a toothed circumferential surface, for example a toothed inner circumferential surface. At least one tooth of the toothed circumferential surface may form the at least one dose stop.

If the dose setting element is configured to set one of a plurality of settable doses, and the body front part comprises a plurality of dose stops, multiple teeth of the toothed circumferential surface may form a plurality of these dose stops.

According to an embodiment, the nut comprises at least one flexible arm configured to interact with the toothed circumferential surface of the body front part by snapping into one of the spaces between two teeth of the toothed circumferential surface to hold the nut in a rotational position relative to the body front part. The nut may comprise multiple, e.g. two or four, flexible arms configured to interact with the toothed circumferential surface of the body front part by each snapping into one of the spaces between two teeth of the toothed circumferential surface to hold the nut in a rotational position relative to the body front part.

According to an embodiment, the toothed circumferential surface forms a linear guide for the nut.

According to one embodiment, the toothed circumferential surface can have multiple functions: it can form one or multiple dose stops, it can form a linear guide for the nut and/or it can form a linear guide for the drive sleeve. Alternatively or additionally, the toothed surface can cooperate with the drive sleeve to rotationally fix the drive sleeve to the body front part.

According to an embodiment, the drive sleeve forms an outer thread meshing with an inner thread of the dose setting sleeve.

According to an embodiment, the medicament delivery mechanism further comprises a maximum dose stop. The maximum dose stop can be an axial or radial maximum dose stop. A radial maximum dose stop may be a maximum dose stop that blocks relative movement in a rotational direction whereas an axial maximum dose stop may be a maximum dose stop that blocks relative movement in an axial direction. A radial maximum dose stop may comprise two stop surfaces being arranged in parallel with a longitudinal axis of the medicament delivery mechanism that are configured to contact each other when a maximum dose is set whereas an axial maximum dose stop may comprise two stop surfaces being arranged obliquely, in particular perpendicular, to a longitudinal axis of the medicament delivery mechanism that are configured to contact each other when a maximum dose is set.

According to an embodiment, the maximum dose stop acts between the dose setting sleeve and the drive sleeve.

According to an embodiment, the drive sleeve forms a stop surface of the maximum dose stop. Alternatively or additionally, the stop surface can be configured to get in contact with the dose setting sleeve when a maximum dose is set.

According to an embodiment, the dose setting sleeve forms a further stop surface of the maximum dose stop.

According to an embodiment, the medicament delivery mechanism comprises a clutch mechanism. The clutch mechanism may rotationally fix the nut to the dose setting sleeve during dose setting and may rotationally decouple the nut from the dose setting sleeve during dose delivery.

For example, the clutch mechanism comprises a coupling member and a coupling element, wherein the coupling member is rotatably fixed to the nut during dose setting and dose delivery, and wherein the dose setting element is rotationally fixed to the dose setting sleeve and the coupling member via said coupling element during dose setting.

According to an embodiment, the dose setting sleeve comprises a marking corresponding to the at least one dose, for example on its outer circumferential surface. The marking may be visible through an opening or a transparent window in the body rear part. The dose sleeve may comprise several markings, each marking corresponding to one of a multitude of settable doses. The marking, such as each marking, may be a number arranged on the outer circumferential surface. For example, multiple markings may be arranged along a helical line.

According to an embodiment, the medicament delivery mechanism comprises a button forming a push surface for delivery of the medicament. Axial movement of the push button relative to the body rear part during dose delivery may cause the dose setting sleeve to rotate due to a threaded connection between the body rear part and the dose setting sleeve. This allows to reset the dose setting sleeve to its initial position during dose delivery.

The button may be coupled to the piston rod during dispensing such that axial movement of the button in a proximal direction results in proximal movement of the piston rod. The button may also form an actuation element of the clutch mechanism that rotationally decouples the nut from the dose setting sleeve during dispensing.

The nut may be configured to rotate with respect to the piston rod during dose setting. Upon dose setting, the nut may be configured to axially move with respect to the piston rod in proportion to the set dose.

During dispensing, i.e. dose delivery, the nut may be configured to remain rotationally stationary with respect to the piston rod. Upon dispensing, the nut may be configured to axially move together with the piston rod without rotation.

The drive sleeve may be configured to axially move together with the nut during dose setting. During dose setting, the nut may be decoupled from the drive sleeve. Axial movement of the drive sleeve and the nut may be independent from each other so that no force is transferred from the drive sleeve to the nut or vice versa. Additionally or alternatively, the nut may rotate with respect to the drive sleeve during dose setting.

During dispensing, the nut and the drive sleeve may move together along the longitudinal axis. The nut may be rotationally fixed with respect to the drive sleeve during dispensing. During dispensing, the drive sleeve may be axially coupled to the nut so that a force is transferred from the drive sleeve to the nut. For example, the drive sleeve may be configured to push the nut in the axial direction, e.g. the proximal direction, during dispensing.

The application is also directed at a medicament delivery device comprising a medicament delivery mechanism according to one of the embodiments mentioned above or below. The medicament delivery device may comprise a medicament container - directly or indirectly - attached or attachable to the medicament delivery mechanism, such as to a housing of the medicament delivery mechanism, for example to the body front part. The medicament container may include a medicament.

According to an embodiment, the medicament delivery device comprises a container holder. The container holder may be configured to hold the medicament container. The medicament container can be connected or connectable to the container holder by a snap fit connection. The container holder can comprise a connection portion to connect the container holder to the medicament delivery mechanism, such as to the housing of the medicament delivery mechanism, for example to the body front part, for example via a snap-fit connection.

The medicament container or the container holder may be non-releasably connected or non-releasably connectable to the medicament delivery mechanism. In other words, the medicament container or container holder may be connected or connectable to the medicament delivery mechanism in a way that a user cannot detach the medicament container or container holder from the medicament delivery mechanism without destroying one or the other. This is preferable if the medicament delivery device is supposed to be discarded after the last dose of the medicament container is delivered. Alternatively, the medicament container or container holder may be releasably connected or releasably connectable to the medicament delivery mechanism, e.g. if the medicament delivery device is a reusable medicament delivery device.

In a second aspect, the present disclosure also relates to a medicament delivery mechanism for dispensing a medicament from a medicament container, the medicament delivery mechanism comprising: a first member, for example a drive sleeve, and a second member, for example a dose setting sleeve or a housing. The first member and the second member are configured to rotate relative to each other during dose setting. The first member and the second member may be configured to axially move relative to each other while they rotate relative to each other during dose setting. The first member comprises at least one first maximum dose stop surface of a maximum dose stop. The second member comprises at least one second maximum dose stop surface of the maximum dose stop. The first and second maximum dose stop surfaces are configured to come in contact with each other when the medicament delivery mechanism is set to a maximum settable dose. The maximum dose stop is configured as a radial stop.

A radial stop blocks relative movement in a rotational direction contrary to an axial stop that blocks relative movement in an axial direction. Therefore, the radial maximum dose stop may comprise two surfaces, such as the first maximum dose stop surface and the second maximum dose stop surface, being generally arranged in parallel with, i.e. along, a longitudinal axis of the medicament delivery mechanism, wherein the two surfaces are configured to contact each other when a maximum dose is set. Contrary thereto, an axial maximum dose stop may comprise two surfaces being arranged obliquely, e.g. perpendicular, to a longitudinal axis of the medicament delivery mechanism, that are configured to contact each other when a maximum dose is set.

Such a radial maximum dose stop allows a precise feedback to the user when the medicament delivery mechanism is set to a maximum dose. Therefore, the radial maximum dose stop decreases the likelihood that a user moves, e.g. turns, a dose setting element past a maximum dose stop. Thereby reliability of the medicament delivery mechanism is improved. Furthermore, a radial maximum dose stop may withstand a higher torque than an axial dose stop. Additionally or alternatively, a radial maximum dose stop may improve accuracy of the dose setting.

The medicament delivery mechanism according to the second aspect of the present disclosure may comprise at least one, several or all features of the medicament delivery mechanism according to the first aspect of the present disclosure. On the other hand, the medicament delivery mechanism according to the first aspect of the present disclosure may comprise at least one, several or all features of the medicament delivery mechanism according to the second aspect of the present disclosure.

According to an embodiment, the at least one radial maximum dose stop surface of the first member is formed at an elastically bendable portion of the first member that is bendable in a radial direction. This can simplify assembly of the medicament delivery mechanism.

According to an embodiment, the elastically bendable portion has a width in the axial direction and a length along a circumferential direction and wherein the length of the elastically bendable portion is greater than the width of the elastically bendable portion. In other words, the elastically bendable portion may extend in the circumferential direction. This allows to develop elastically bendable portions that bend more easily due to being relatively long.

According to an embodiment, the elastically bendable portion has a free end with a front surface forming a radial front surface.

According to an embodiment, the radial front surface at the free end is configured as the first maximum dose stop surface.

According to an embodiment, at least one of the first maximum dose stop surface and the second maximum dose stop surface, such as both the first maximum dose stop surface and the second maximum dose stop surface, form oblique surfaces to force the first member, e.g. the elastically bendable portion of the first member, towards the second member when the first maximum dose stop surface and the second maximum dose stop surface come in contact with each other. This feature allows to increase a force needed to move the dose setting element past the maximum dose position while at the same time to decrease a force needed for assembly, e.g. of the first member and the second member.

According to an embodiment, the radial front surface of the elastically bendable portion forms an oblique surface to force the elastically bendable portion towards the second member when the first maximum dose stop surface and the second maximum dose stop surface come in contact with each other.

According to an embodiment, the first maximum dose stop surface and/or the second maximum dose stop surface is positioned in a plane that is spaced apart from a centerline of the medicament delivery mechanism. In other words, the first maximum dose stop surface and/or the second maximum dose stop surface can be arranged in a way that they do not lie or partly do not lie in a radial plane. For example, sections of or the whole first maximum dose stop surface and/or sections of or the whole second maximum dose stop surface may be arranged obliquely to a radial plane and/or planes comprising the centerline.

For example, sections of the first maximum dose stop surface or the whole first maximum dose stop surface may lie in a first plane that is offset from the longitudinal axis of the first member. The longitudinal axis of the first member may be a rotation axis of the first member for rotation with respect to the second member.

For example, sections of the second maximum dose stop surface or the whole second maximum dose stop surface may lie in a second plane that is offset from the longitudinal axis of the second member. The longitudinal axis of the second member may be a rotation axis of the second member for rotation with respect to the first member. The longitudinal axis of the first member may coincide with the longitudinal axis of the second member. Additionally or alternatively, the longitudinal axis of the first member and/or the longitudinal axis of the first member may coincide with the centreline of the medicament delivery mechanism.

According to an embodiment, the first maximum dose stop surface and the second maximum dose stop surface are at least in sections formed to interlock with each other. For example, the first maximum dose stop surface can be shaped in a convex way and the corresponding second maximum dose stop surface can be shaped in a concave way or vice versa. Generally, the first maximum dose stop surface can form a recess and the corresponding second maximum dose stop surface can form an elevation or vice versa. Such a non-flat overall shape of the first and second maximum dose stop surfaces allows the first and second maximum dose stop surfaces to interlock with each other. This limits the risk of the first and second maximum dose stop surfaces pushing each other out of the way when a user tries to moves the dose setting element past the maximum dose position.

According to an embodiment, the first maximum dose stop surface and the second maximum dose stop surface can be configured to displace a part of one of the first member and the second member in the direction towards the other member of the first member and the second member. For example, the first maximum dose stop surface and/or the second maximum dose stop surface can comprise obliquely arranged surfaces in a cross-sectional view perpendicular to the longitudinal axis of the medicament delivery mechanism that force an outer circumferential surface of the first member onto an inner circumferential surface of the second member when the first member and the second member are rotated relative to each other past their maximum dose positions. This concept has the advantage that higher forces applied by the user to move the dose setting member past the maximum dose position result in higher friction between the first member and the second member.

According to an embodiment, the medicament delivery mechanism comprises a plurality of radial maximum dose stops, such as two maximum dose stops. Each dose stop may comprise a respective first maximum dose stop surface provided at the first member and a respective second maximum dose stop surface provided at the second member. This allows to reduce the force that needs to be transferred by each dose stop, i.e. by each corresponding pair of first radial maximum dose stop surfaces and second radial maximum dose stop surfaces.

According to an embodiment, the plurality of radial maximum dose stops are distributed equally around the circumference of the first member and/or the second member. For example, the medicament delivery mechanism may comprise two radial maximum dose stops arranged 180° apart from each other. The first member can comprise two first radial maximum dose stop surfaces arranged 180° apart from each other and the second member can comprise two second radial maximum dose stop surfaces arranged 180° apart from each other.

According to an embodiment, the first member is at least partly arranged inside the second member.

According to an embodiment, the first member extends through a generally cylindrical portion of the second member. More generally, the first member may comprise a section that is located inside a space that is at least partly surrounded by the second member. According to an embodiment, the first member comprises a cylindrical portion that is arranged inside a cylindrical portion of the second member.

According to an embodiment, the first member is linearly guided and rotationally fixed relative to a housing of the medicament delivery mechanism and/or the second member is threadedly coupled to a housing of the medicament delivery mechanism.

According to an embodiment, the first member is configured to transfer an axial force via a nut to a piston rod during dose delivery. For example, the nut engages with the piston rod and abuts the first member to transfer the axial force from the first member to the nut and from the nut to the piston rod during dose delivery.

According to an embodiment, the first member is configured to transfer an axial force from the second element to the nut during dose delivery. For example, the first member engages with and/or abuts the second member and/or the nut to transfer the axial force from the second member to the nut during dose delivery.

According to an embodiment, the first member forms a first thread and the second member forms a second thread engaging the first thread. In other words, the first member and the second member can be threadedly engaged with each other.

According to an embodiment, the first maximum dose stop surface is spaced apart from an end surface of the first thread and/or the second maximum dose stop surface is spaced apart from an end surface of the second thread. In other words, the first maximum dose stop surface may not be an end surface of the first thread and/or the second maximum dose stop surface may not be an end surface of the second thread.

According to a more general embodiment, the first maximum dose stop surface does not form a delimiting surface of the first thread and/or the second maximum dose stop surface does not form a delimiting surface of the second thread.

According to an embodiment, the first maximum dose stop surface is configured separate from the first thread and/or the second maximum dose stop surface is configured separate from the second thread.

According to an embodiment, the first thread is a single-start thread and the second thread is a single-start thread. In other words, for example, the first thread is not a multi-start thread and the second thread is not a multi-start thread.

According to an embodiment, a number of the radial maximum dose stops is larger than a number of starts of the first thread and a number of starts of the second thread. For example, there are two radial maximum dose stops and the first thread and second thread are single-start threads.

According to an embodiment, the first member is a single integral part. For example, the first member is completely made from the same material and/or manufactured in one manufacturing step, for example by molding, such as injection molding. This makes it easier to assemble the medicament delivery mechanism.

For example, the first maximum dose stop surface is formed integrally with the first member.

According to an embodiment, the second member is a single integral part. For example, the second member is completely made from the same material and/or manufactured in one manufacturing step, for example by molding, such as injection molding. This makes it easier to assemble the medicament delivery mechanism.

For example, the second maximum dose stop surface is formed integrally with the second member.

According to an embodiment, the at least one first radial maximum dose stop surface is arranged at a distal end portion of the first member located at a distal end of the first member. The distal end of the first member can be the opposite end of where the first member abuts the nut. Hence, the first member may abut the nut with its proximal end.

According to an embodiment, the medicament delivery mechanism comprises a housing. The housing may comprise a body front part and a body rear part. The body front part and the body rear part may form separate members that are fixedly connected to each other, e.g. by a snap-fit connection.

According to an embodiment, the housing, e.g. the body front part, forms a linear guide for the first member that rotationally fixes the first member to the housing. Additionally or alternatively, the housing, e.g. the body rear part, may form a thread that meshes with a thread of the second member.

According to an embodiment, the medicament delivery mechanism comprises a piston rod extending from the housing, e.g. the body front part, for advancing a plunger within the medicament container to deliver the medicament.

According to an embodiment, the nut forms a threaded connection with the piston rod.

According to an embodiment, the housing, e.g. the body front part, forms a linear guide for the nut. According to an embodiment, said linear guide for the nut is configured to additionally be a linear guide for the drive sleeve.

According to an embodiment, the nut comprises at least one flexible arm configured to interact with a toothed inner circumferential surface of the housing, in particular the body front part, by snapping into one of the spaces between two teeth of the toothed inner circumferential surface to hold the nut in a rotational position relative to the housing, e.g. the body front part. The nut may comprise multiple, e.g. four, flexible arms configured to interact with the toothed circumferential surface of the housing, e.g. the body front part, by each snapping into one of the spaces between two teeth of the toothed circumferential surface to hold the nut in a rotational position relative to the housing, e.g. the body front part.

According to an embodiment, the toothed inner circumferential surface forms the linear guide for the nut.

According to an embodiment, the housing, e.g. the body front part, forms a container connector to - directly or indirectly - connect the medicament container to the housing, e.g. the body front part.

According to an embodiment, the container connector is a snap-fit connector, such as a non-releasable snap-fit connector.

According to an embodiment, the body front part and the body rear part form corresponding and mutual engaging body connectors to connect the body front part and the body rear part. The body connectors may be configured as snap-fit connectors.

According to an embodiment, the body front part is at least partly disposed within the body rear part.

According to an embodiment, the body rear part forms an outer housing defining an outer surface of the medicament delivery mechanism.

According to an embodiment, the body front part forms an outer surface of the medicament delivery device, the outer surface being, for example, located proximally from the outer surface formed by the body rear part.

According to an embodiment, the linear guide for the first member formed by the housing, e.g. by the body front part, comprises a toothed inner circumferential surface.

According to an embodiment, the housing, e.g. the body front part, forms a linear guide for the piston rod that rotationally fixes the piston rod to the housing, e.g. the body front part. For example, the housing, e.g. body front part, forms a non-round, e.g. oval, opening and the piston rod forms a corresponding non-round, e.g. oval, outer shape.

According to an embodiment, the housing, e.g. the body rear part, forms a threaded connection with the second member, e.g. the dose setting sleeve.

According to an embodiment, the medicament delivery mechanism comprises a clutch mechanism. The clutch mechanism may rotationally fix the nut to the second member, e.g. the dose setting sleeve, during dose setting and may rotationally decouple the nut from the second member, e.g. the dose setting sleeve, during dose delivery.

According to an embodiment, the medicament delivery mechanism comprises a dose setting element for setting at least one dose. The second member may be rotationally fixed to the dose setting element during dose setting.

According to an embodiment, the clutch mechanism comprises a coupling element and a coupling member. The coupling member may be rotatably fixed to the nut during dose setting and dose delivery. The dose setting element may be rotationally fixed to the second member, e.g. the dose setting sleeve, and the coupling member via said coupling element during dose setting.

According to an embodiment, the second member, e.g. the dose setting sleeve, comprises a marking corresponding to the at least one dose on its outer circumferential surface and wherein the marking is visible through an opening or a transparent window in the housing, in particular the body rear part. The marking can comprise multiple numbers arranged along the outer circumferential surface, e.g. along a helical line.

According to an embodiment, the medicament delivery mechanism comprises a button forming a push surface for delivery of the medicament. Axial movement of the push button relative to the housing, e.g. the body rear part, during dose delivery may cause the second member, e.g. the dose setting sleeve, to rotate due to a threaded connection between the housing, e.g. the body rear part, and the second member, e.g. the dose setting sleeve.

Generally, the features described in relation to the maximum dose stop could be applied instead or additionally to a last dose stop and/or a zero dose stop. A last dose stop prevents setting of a dose that is larger than a remaining amount of medicament in the container. The last dose stop may act between the piston rod and the nut. For example, the piston rod may form the first member and the nut may form the second member or vice versa. A zero dose stop defines the zero dose position of the medicament delivery mechanism, such as a zero dose position of the dose sleeve. The zero dose stop may act between the drive sleeve and the dose setting sleeve. For example, the drive sleeve may form the first member and the dose setting sleeve may form the second member or vice versa. Alternatively, the zero dose stop may act between the dose setting sleeve and the housing. The dose setting sleeve then may form the first member and the housing may form the second member or vice versa.

The application is also directed at a medicament delivery device comprising a medicament delivery mechanism according to one of the abovementioned or below mentioned embodiments, and a medicament container attached to the housing, e.g. the body front part. The medicament container may include a medicament.

According to an embodiment, the medicament delivery device further comprises a container holder. The medicament container may be held within the container holder, for example by a snap fit connection.

The devices and mechanisms according to the present disclosure may be configured to dispense a drug or, used synonymously herein, a medicament.

According to the present disclosure, a drug or a medicament is a pharmaceutical formulation that contains one or more active pharmaceutical ingredients. An active pharmaceutical ingredient is a substance that is biologically active or has a biological effect on humans or animals. It is used in the diagnosis, mitigation, cure, treatment or prevention of a medical condition or to otherwise affect the structure or function of the body of humans or animals.

Active pharmaceutical ingredients, medicaments and drugs that may be contained and/or dispensed by the mechanisms and devices according to the present disclosure are, inter alia, described in handbooks such as Merck Index, 15th edition, and Rote Liste 2023. Both handbooks are freely accessible via the Internet.

Active pharmaceutical ingredients, medicaments and drugs that may be contained and/or dispensed by the mechanisms and devices according to the present disclosure may be selected from: analgesics (main group 05 of Rote Liste); anti-obesity drugs (main group 06 of Rote Liste); antiallergics (main groups 07 of Rote Liste); antianemics (main group 08 of Rote Liste); antidiabetic drugs (main group 12 of Rote Liste); antidote medications (main group 13 of Rote Liste); antihemorrhagics (antifibrinolytics and other hemostatic agents) (main group 16 of Rote Liste); anti hypoglycemic drugs (main group 18 of Rote Liste); antihypotonics and agents for shock therapy (main group 19 of Rote Liste); anticoagulants (main group 20 of Rote Liste); angiotensin-aldosterone system (main group 27 of Rote Liste); bronchospasmolytics/antiasthmatics and other agents for the respiratory tract (main group 28 of Rote Liste); dermatics (main group 32 of Rote Liste); diagnostic agents and agents for diagnostic preparation (main group 35 of Rote Liste); enzyme inhibitors, preparations for enzyme deficiency and transport proteins (main group 40 of Rote Liste); gene and cell therapeutics and RNA interference therapeutics (main group 42 of Rote Liste); hypophysial and hypothalamic hormones, other regulatory peptides, their inhibitors and analogues (main group 50 of Rote Liste); immunomodulators (main group 51 of Rote Liste); lipid-lowering drugs (main group 58 of Rote Liste); migraine medication (main group 61 of Rote Liste); neuropathy preparations and other neurotropic agents (main group 66 of Rote Liste); ophthalmics (main group 67 of Rote Liste); osteoporosis agents, calcium/bone metabolism regulators (main group 68 of Rote Liste); psychotropic drugs (main group 71 of Rote Liste); sera, immunoglobulins and vaccines (main group 75 of Rote Liste); sex hormones and their inhibitors (main group 76 of Rote Liste); vitamins (main group 84 of Rote Liste); oncology drugs, cytostatics, other antineoplastic agents and protectives (main group 86 of Rote Liste); and anaesthetics.

The drug or medicament may have, for example, one or more active pharmaceutical ingredients selected from one or more of: carbohydrates and polysaccharides; polypeptides, peptides and proteins (e.g., antibodies, antibody fragments, hormones, growth factors, and enzymes); small molecules with a molecular weight of 500 Da or less; and nucleic acids, double or single stranded DNA (including cDNA and naked DNA), RNA, antisense nucleic acids, such as antisense DNA and RNA, small interfering RNA (siRNA), ribozymes, genes, and oligonucleotides. Nucleic acids may be included into molecular delivery systems such as liposomes, vectors, or plasmids.

The drugs or medicaments contained in or dispensed with the mechanisms according to the present disclosure may comprise active pharmaceutical ingredients used in the treatment and/or mitigation and/or prevention and/or cure and/or diagnosis of many different types of medical conditions. These may include, inter alia, one or more of the following conditions: acute coronary syndrome, angina, myocardial infarction, anaphylactic shock, atherosclerosis, diabetes mellitus or complications associated with type 1 or type 2 diabetes mellitus such as diabetic retinopathy, cancer, macular degeneration, inflammation, hay fever, rheumatoid arthritis, thromboembolism disorders such as deep vein or pulmonary thromboembolism, infertility, and growth disorder.

For example, the active pharmaceutical ingredients for the therapy of type 1 or type 2 diabetes mellitus or complications associated with type 1 or type 2 diabetes mellitus include an insulin, such as, human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof.

The active pharmaceutical ingredient may also be a hormone, such as a hypophysis hormone or a hypothalamus hormone or a regulatory active peptide and an antagonist of a regulatory active peptide. The hormone may be a fertility hormone, a gonadotropine, a growth hormone, a steroid hormone, such as testosterone or oestrogen, a parathyroid hormone, such as teriparatide, epinephrine or a follicle-stimulating hormone.

The active pharmaceutical ingredient may also be an oligonucleotide.

The active pharmaceutical ingredient may also be an antibody. The term "antibody" includes an immunoglobulin molecule or an antigen-binding portion, such as a fragment antigen-binding region, of an immunoglobulin molecule, an antigen-binding molecule based on tetravalent bispecific tandem immunoglobulins, and/or a dual variable region antibody-like binding protein having cross-over binding region orientation (CODV). The antibody may be a monoclonal, a polyclonal, a recombinant, or a chimeric antibody. It may be a de-immunized or humanized, a fully human, or a non-human, such as a murine, antibody. The antibody may be a single chain antibody.

The active pharmaceutical ingredient may also be a polysaccharide, such as a glucosaminoglycane, a hyaluronic acid, a heparin, a low molecular weight heparin or an ultra-low molecular weight heparin or a derivative thereof. The active pharmaceutical ingredient may also be a sulphated polysaccharide, e.g. a poly-sulphated form of the above-mentioned polysaccharides, and/or a pharmaceutically acceptable salt thereof.

The drug or medicament contained in and/or dispensed with the mechanisms and devices according to the present disclosure may include one or more of the following active pharmaceutical ingredients: abatacept, adalimumab, aflibercept, alirocumab, anakinra, apomorphine, aripiprazole, belimumab, benralizumab, betamethasone, bimekizumab, brodalumab, brolucizumab, buprenorphine, certolizumab pegol, choriogonadotropin alfa, cyanocobalamin, carbepoetin alfa, denosumab, dulaglutide, dupilumab, enoxaparin sodium, epinephrine, epoetin, epoetin alfa, epoetin beta, epoetin theta, epoetin zeta, erenumab, etanercept, evolocumab, exenatide, filgrastim, follitropin, follitropin alfa, follitropin delta, folic acid, fondaparinux, fremanezumab, fulvestrant, gadobenic acid, gadobutrol, gadodiamide, gadoteric acid, gadoxetic acid, galcanezumab, ganirelix, glatiramer, glucagon, golimumab, goserelin, guselkumab, heparin, icatibant, inclisiran, infliximab, inotersen, insulin aspart, insulin degludec, insulin detemir, insulin glargine, insulin glulisine, insulin human, insulin human-isophane, insulin lispro, insulin isophane, interferon beta-1a, ixekizumab, lanadelumab, lanreotide, lebrikizumab, leuprorelin, liraglutide, lonapegsomatropin, lutropin alfa, menotropin, mepolizumab, methotrexate, methoxy-polyethylene glycol-epoetin beta, mirikizumab, moroctocog alfa, natalizumab, nirsevimab, ofatumumab, omalizumab, paliperidone, pegfilgrastim, peginterferon alfa-2a, peginterferon beta-1a, pegvaliase, propofol, pyridoxine, ranibizumab, rho(D) immune globulin, risankizumab, romosozumab, ropeginterferon alfa-2b, sarilumab, satralizumab, secukinumab, semaglutide, somapacitan, somatrogon, somatropin, sumatriptan, teriparatide, tezepelumab, tildrakizumab, tinzaparin sodium, tocilizumab, tralokinumab, triptorelin, ustekinumab, vedolizumab, volanesorsen, and vutrisiran.

Additionally or alternatively, the drug or medicament contained in and/or dispensed with the mechanisms and devices according to the present disclosure may include one or more of the following active ingredients: Lixisenatide, Taspoglutide, Albiglutide, Efpeglenatide, GLP-1 Eligen, Nodexen, Pegapamodtide, Tirzepatide, and Bamadutide.

Additionally or alternatively, the drug or medicament dispensed with the mechanisms and devices according to the present disclosure may include one or more of the following active ingredients: Mipomersen sodium, Linagliptin, Vildagliptin, Sitagliptin, Denagliptin, Saxagliptin, Berberine, Lutropin, Choriongonadotropin, Desmopressin, Terlipressin, Gonadorelin, Buserelin, Nafarelin, Hylan G-F 20.

The drug or medicament may also contain one or more of the following: pharmaceutically acceptable solvents or salts of the active pharmaceutical ingredients described herein; analogues and derivates of the active pharmaceutical ingredients described herein; biosimilars of the active pharmaceutical ingredients described herein; and pharmaceutically acceptable carriers of the active pharmaceutical ingredients described herein.

The drug or medicament contained in and/or dispensed with the mechanisms and devices according to the present disclosure may include one or more of the following active pharmaceutical ingredients: paracetamol (acetaminophen), nonsteroidal anti-inflammatory drugs, such as aspirin, ibuprofen and naproxen, cox-2 inhibitors, such as rofecoxib, celecoxib, and etoricoxib, opioids, such as morphine, codeine, oxycodone, hydrocodone, dihydromorphine, pethidine, alcohols, cannabis, nefopam, flupirtine, ziconotide.

The drug or medicament may be contained in a container from which it is dispensed by an action of a user. The container may, for example, be a cartridge, a reservoir or a syringe. The container may extend between a dispensing end and a rear end of the container. The container may be configured to dispense the drug or medicament at the dispensing end, for example through a pierceable septum. At the rear end, the container may be sealed to prevent the exit of the drug or medicament. For example, the container may be sealed by a flexible and/or displaceable plunger at the rear end. In between the dispensing end and the rear end, the container may have a generally tubular hollow housing.

The container may have a single compartment for storing the drug or medicament. Alternatively, the container may also have more than one compartment, such as two compartments. Different compartments thereby may comprise different substances, such as drugs, medicaments, active ingredients, solvents or carriers. Different compartments may also comprise a part of the medicament or drug in solid form, for example as a powder, such as a lyophilized powder, and another part of the medicament in liquid form. The solid part may, for example, comprise the one or more active pharmaceutical ingredient and/or the liquid part may be a solvent.

The container may be configured to allow a mixing of the contents from at least two of the compartments, such as from all of the compartments, prior to and/or during dispensing of the medicament or drug. For example, the container may be configured to provide a fluid connection between the at least two, such as between all of the compartments, for mixing.

For example, the container may comprise two compartments, one comprising a first part of the drug or medicament in solid form, for example the one or more active ingredients, and the other one comprising a second part of the drug or medicament in liquid form, such as a solvent. The container may be configured to provide a fluid connection between the two compartments and to mix the first and second parts prior or during dispensing. This may reconstitute the drug or medicament from its solid form.

The devices and mechanisms according to the present disclosure may be disposable, which means that they are disposed of after having dispensed a last dose from the container. Alternatively, they may be reusable, for example, they may allow to replace a container from which the last dose has been dispensed by a new container or to refill a container from which the last dose has been dispensed.

The devices and mechanisms according to the present disclosure may be fixed dose devices and mechanisms that only allow the dispensing of a fixed dose of the medicament or drug. Alternatively, they may be variable dose devices and mechanisms that allow for setting of a dose from a multitude of differing settable doses.

The mechanisms and devices according to the present disclosure may comprise a dose definition mechanism that allows a user to set the dose to be dispensed prior to dispensing. The dose thereby may be settable as a fixed dose only, for example for fixed dose devices or mechanisms. The dose also may be settable from a multitude of settable doses, such as for variable dose devices or mechanisms.

The doses may be settable as integer multiples of international units of the drug or medicament. They also may be settable as integer multiples of a fraction of said international units, such as integer multiples of half units.

The mechanisms and devices according to the present disclosure may be single dose devices that allow dispensing of a dose from the container only once, so that the container is a single dose container. They also may be multiple dose devices that allow for a sequential dispensing of more than one dose from the same container, so that the container is a multi-dose container.

The drug delivery devices according to the present disclosure may be injection devices, such as needle-based injection devices.

### DRAWINGS

Exemplary embodiments and functions of the present disclosure are described herein in conjunction with the following drawings, showing schematically:
- Fig. 1: an exploded view of a medicament delivery device according to the present disclosure having a medicament delivery mechanism according to a first embodiment of the present disclosure;
- Fig. 2A: a first perspective view of a cap of the medicament delivery device of Fig. 1;
- Fig. 2B: a second perspective view of the cap of Fig. 2A;
- Fig. 3A: a side view of the cap of Fig. 2A;
- Fig. 3B: a bottom view of the cap of Fig. 2A;
- Fig. 3C: a cross-sectional side view of the cap of Fig. 2A along the line BE-BE of Fig. 3A;
- Fig. 4: a perspective view of a container holder of the medicament delivery device of Fig. 1;
- Fig. 5A: a side view of the container holder of Fig. 4;
- Fig. 5B: a cross-sectional side view of the container holder of Fig. 4 along the line N-N of Fig. 5A;
- Fig. 5C: a bottom view of the container holder of Fig. 4;
- Fig. 5D: a top view of the container holder of Fig. 4;
- Fig. 6A: a perspective view of a medicament container of the medicament delivery device of Fig. 1;
- Fig. 6B: a side view of the medicament container of Fig. 6A;
- Fig 6C: a cross-sectional side view of the medicament container of Fig. 6A;
- Fig. 6D: a bottom view of the medicament container of Fig. 6A;
- Fig. 6E: a top view of the medicament container of Fig. 6A;
- Fig. 7A: a first perspective view of a piston of the medicament delivery device of Fig. 1 ;
- Fig. 7B: a second perspective view of the piston of Fig. 7A;
- Fig. 8A: a side view of the piston of Fig. 7A;
- Fig. 8B: a cross-sectional side view of the piston of Fig. 8A along the line P-P;
- Fig 8C: a top view of the piston of Fig. 8A;
- Fig. 8D: a bottom view of the piston of Fig. 8A;
- Fig. 9A: a perspective view of a piston rod of the medicament delivery device of Fig. 1;
- Fig. 9B: a side view of the piston rod of Fig. 9A;
- Fig. 9C: a cross-sectional side view of the piston rod of Fig. 9A along the line M-M of Fig. 9B;
- FIG. 9D: a bottom view of the piston rod of Fig. 9A;
- Fig. 9E: a top view of the piston rod of Fig. 9A;
- Fig. 10A: a first perspective view of a body front part of the medicament delivery device of Fig. 1;
- Fig. 10B: a second perspective view of the body front part of the medicament delivery device of Fig. 1;
- Fig. 11A: a side view of the body front part of Fig. 10A;
- Fig. 11B: a cross-sectional side view of the body front part of Fig. 11A along the line AA-AA of Fig. 11A;
- Fig. 11C: a cross-sectional view of the body front part of Fig. 11A along the line AB-AB of Fig. 11A;
- Fig. 11D: a top view of the body front part of Fig. 11A;
- Fig 12A: a first perspective view of a body rear part of the medicament delivery device of Fig. 1;
- Fig. 12B: a second perspective view of the body rear part of the medicament delivery device of Fig. 1;
- Fig. 13A: a side view of the body rear part of Fig. 12A;
- Fig. 13B: a cross-sectional side view of the body rear part of Fig. 13A along the line K-K of Fig. 13A;
- Fig. 13C: a cross-sectional view of the body rear part of Fig. 13A along the line D-D of Fig. 13A;
- Fig. 14A: a first perspective view of a nut of the medicament delivery device of Fig. 1;
- Fig. 14B: a side view of the nut of Fig. 14A;
- Fig. 14C: a cross-sectional side view of the nut of Fig. 14A along the line E-E of Fig. 14B;
- Fig. 14D: a bottom view of the nut of Fig 14B;
- Fig. 14E: a top view of the nut of Fig. 14B;
- Fig. 15A: a first perspective view of a drive sleeve of the medicament delivery device of Fig. 1;
- Fig. 15B: a side view of the drive sleeve of Fig. 15A;
- Fig. 15C: a cross-sectional side view of the drive sleeve of Fig. 15A along the line G-G of Fig. 15B;
- Fig. 15D: a cross-sectional view of the drive sleeve of Fig. 15A along the line F-F of Fig. 15B;
- Fig 15E: a top view of the drive sleeve of Fig. 15A;
- Fig. 16A: a first perspective view of a dose setting sleeve of the medicament delivery device of Fig. 1;
- Fig. 16B: a second perspective view of the dose setting sleeve of the medicament delivery device of Fig. 1;
- Fig. 17A: a side view of the dose setting sleeve of Fig. 16A;
- Fig. 17B: a cross-sectional side view of the dose setting sleeve of Fig. 17A along the line J-J of Fig. 17A;
- Fig. 17C: a top view of the dose setting sleeve of Fig. 17A;
- Fig. 17D: a cross-sectional view of the dose setting sleeve of Fig. 17A along the line H-H of Fig. 17A;
- Fig. 18A: a perspective view of a biasing member of the medicament delivery device of Fig. 1;
- Fig. 18B: a top view of the biasing member of Fig. 18A;
- Fig. 18C: a side view of the biasing member of Fig. 18A;
- Fig. 19: a perspective view of a coupling member of the medicament delivery device of Fig. 1;
- Fig. 20A: a side view of the coupling member of Fig. 19;
- Fig. 20B: a cross-sectional side view of the coupling member of Fig. 19 along the line A1-A1 of Fig. 20A;
- Fig. 20C: a cross-sectional view of the coupling member of Fig. 19 along the line A-A of Fig. 20A;
- Fig. 20D: a top view of the coupling member of Fig. 20A;
- Fig. 21A: a first perspective view of a coupling element of the medicament delivery device of Fig. 1;
- Fig. 21B: a second perspective view of the coupling element of the medicament delivery device of Fig. 1;
- Fig. 22A: the coupling element of Fig. 21A;
- Fig. 22B: a top view of the coupling element of Fig. 22A;
- Fig. 22C: a cross-sectional view of the coupling element of Fig. 22A along the line H-H of Fig. 22B;
- Fig. 22D: a cross-sectional view of the coupling element of Fig. 22A along the line J-J of Fig. 22B;
- Fig. 23A: a perspective view of a washer of the medicament delivery device of Fig. 1;
- Fig. 23B: a cross-sectional view of the washer of Fig. 23A along the line c-c of Fig. 23C;
- Fig. 23C: a top view of the washer of Fig. 23A;
- Fig. 24A: a perspective view of a bearing of the medicament delivery device of Fig. 1;
- Fig. 24B: a top view of the bearing of Fig. 24A;
- Fig. 24C: a cross-sectional view of the bearing of Fig. 24B along the line B-B of Fig. 24B;
- Fig. 25A: a first perspective view of a dose setting element of the medicament delivery device of Fig. 1;
- Fig. 25B: a second perspective view of the dose setting element of the medicament delivery device of Fig. 1;
- Fig. 26A: a side view of the dose setting element of Fig. 25A;
- Fig 26B: a cross-sectional view of the dose setting element of Fig. 26A along the line AD-AD of Fig. 26A;
- Fig. 26C: a top view of the dose setting element of Fig. 25A;
- Fig. 27A: a first perspective view of a button of the medicament delivery device of Fig. 1 ;
- Fig. 27B: a second perspective view of the button of the medicament delivery device of Fig. 1;
- Fig. 28A: a side view of the button of Fig. 27A;
- Fig. 28B: a top view of the button of Fig. 28A;
- Fig. 28C: a cross-sectional side view of the button of Fig. 27A along the line T-T of Fig. 28B;
- Fig. 29: the medicament delivery device of Fig. 1 in an assembled state;
- Fig. 30A: the medicament delivery device of Fig. 1 in a zero-dose-state;
- Fig. 30B: a cross-sectional view of the delivery device of Fig. 30A along the line A-A of Fig. 30A;
- Fig. 30C: the medicament delivery device of Fig. 1 in a set-dose-state;
- Fig. 30D: a cross-sectional view of the delivery device of Fig. 30C along the line A-A of Fig. 30C;
- Fig. 31A: the medicament delivery device of Fig. 1 in a knob-pressed-state;
- Fig. 31B: a cross-sectional view of the delivery device of Fig. 31A along the line B-B of Fig. 31A;
- Fig 32A: a side view of an alternative dose setting sleeve for a medicament delivery device as shown in Fig. 1;
- Fig. 32B: a cross-sectional side view of the dose setting sleeve of Fig. 32A along the line A-A of Fig. 32A;
- Fig. 32C: a cross-sectional side view of the dose setting sleeve of Fig. 32A along the line B-B of Fig. 32A;
- Fig. 33A: a first perspective view of an alternative drive sleeve corresponding to the dose setting sleeve of Fig. 32A for a medicament delivery device like Fig. 1;
- Fig. 33B: a second perspective view of the alternative drive sleeve of Fig. 33A;
- Fig. 33C: a side view of the alternative drive sleeve of Fig. 33A;
- Fig. 33D: a cross-sectional view of the alternative drive sleeve of Fig. 33A along the line A-A of Fig. 33C;

- Fig. 34A: a side view of an assembly of the dose setting sleeve of Fig. 32A and the drive sleeve of Fig. 33A in a maximum dose position;
- Fig. 34B: a cross-sectional side view of the assembly of Fig. 34A along the line A-A of Fig. 34A; and
- Fig. 34C: a cross-sectional view of the assembly of Fig. 34A along the line B-B of Fig. 34A.

### DETAILED DESCRIPTION

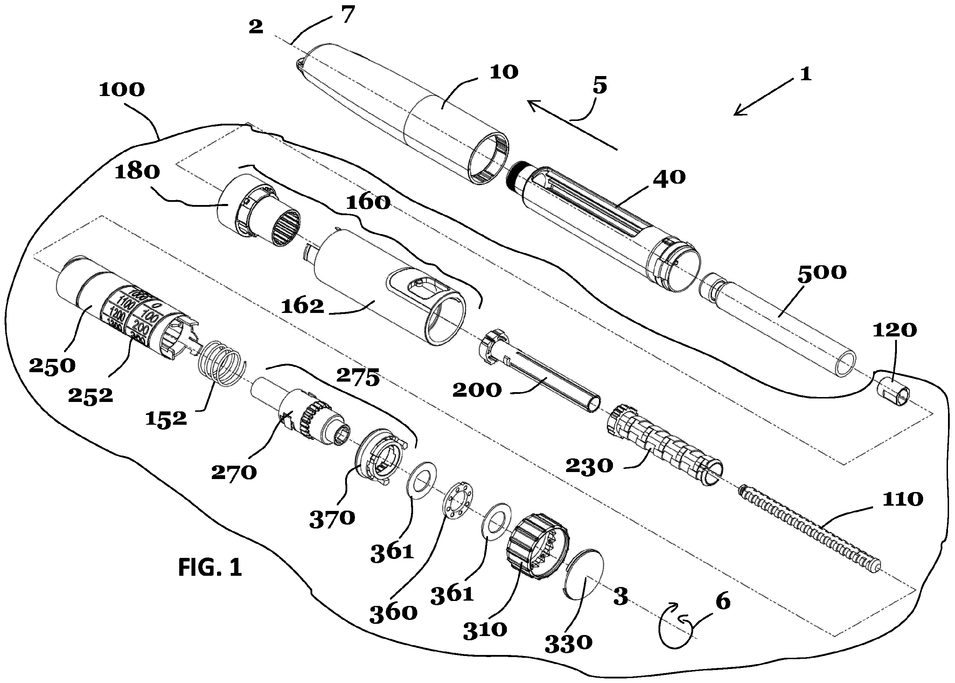

Fig. 1 depicts an exploded perspective view of a medicament delivery device 1 including a medicament delivery mechanism 100. The medicament delivery device 1 has a proximal end 2 and a distal end 3. Starting at the proximal end 2, the medicament delivery device 1 comprises a cap 10, a container holder 40, a medicament container 500, a piston 120, a housing 160 that includes a body front part 180 and a body rear part 162, a nut 200, a drive sleeve 230, a piston rod 110, a dose setting sleeve 250, a biasing member 152, a clutch mechanism 275 that includes a coupling member or connector 270 and a coupling element 370, two washers 361, one of them arranged on a proximal and distal side of a bearing 360, a dose setting element 310, and a button 330. The medicament delivery device 1 is configured as an injection device, namely as a pen injection device.

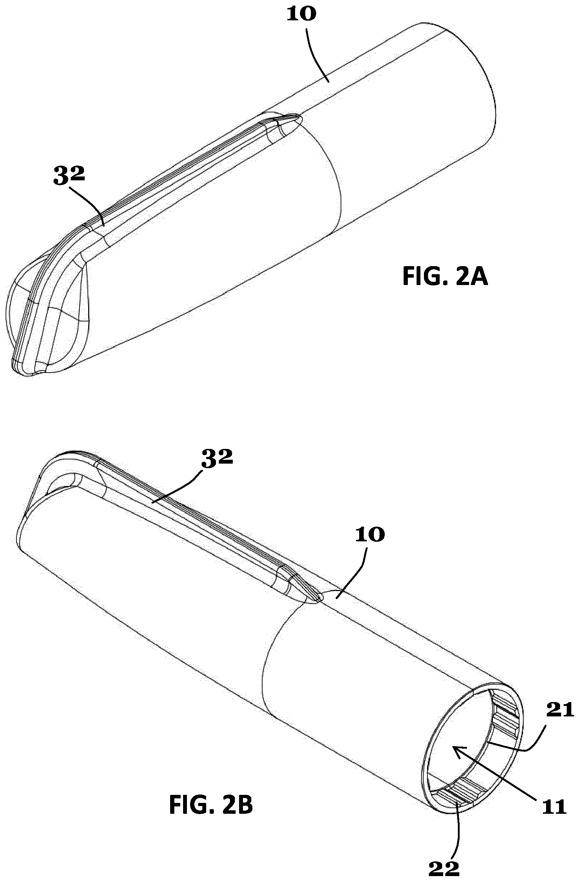

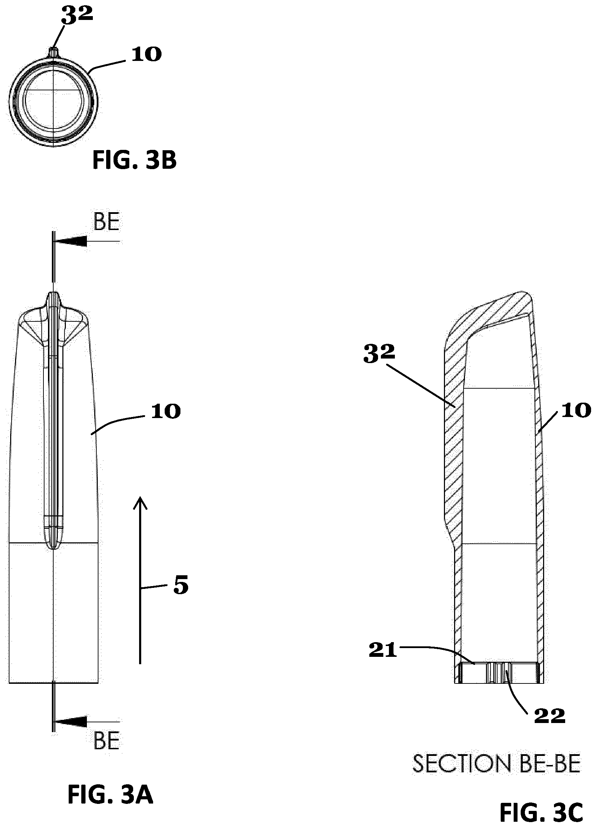

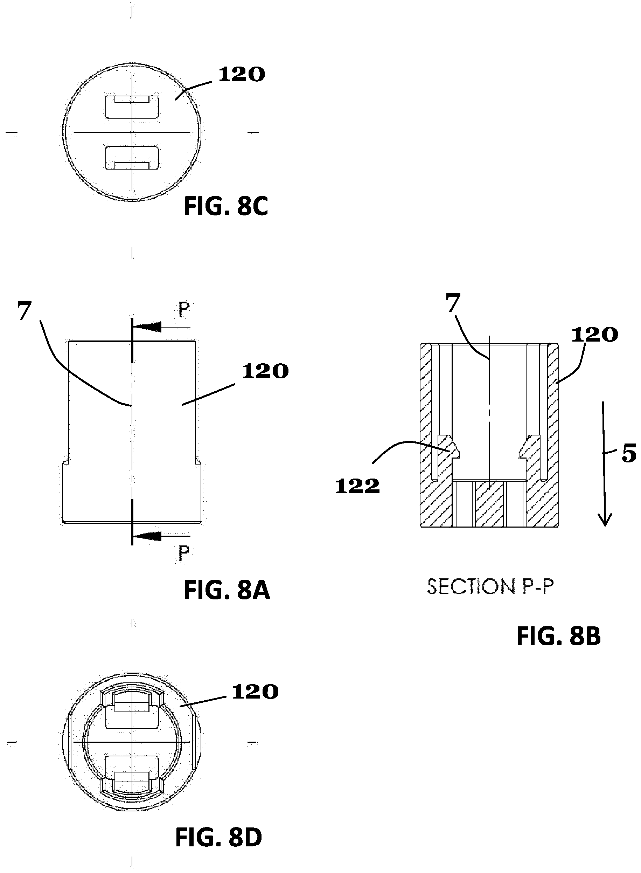

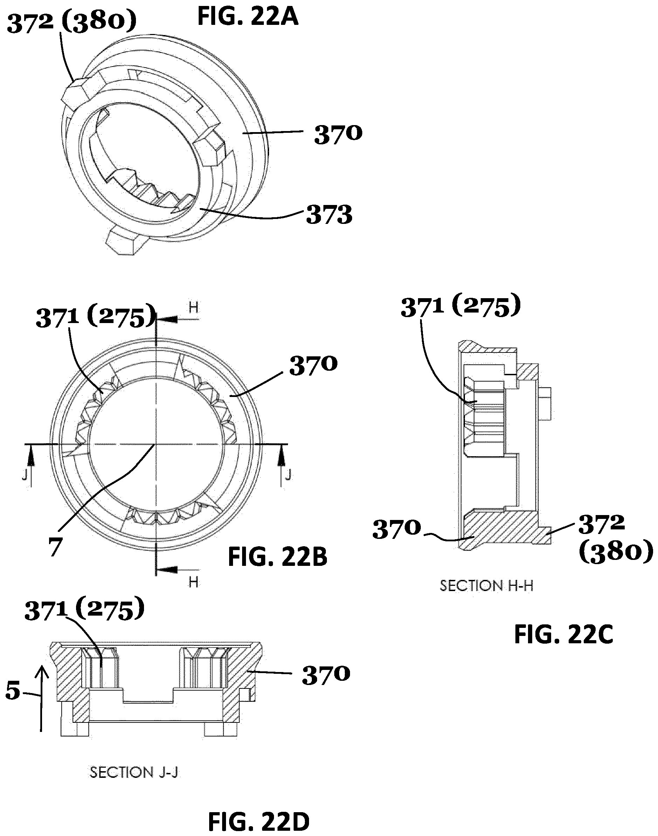

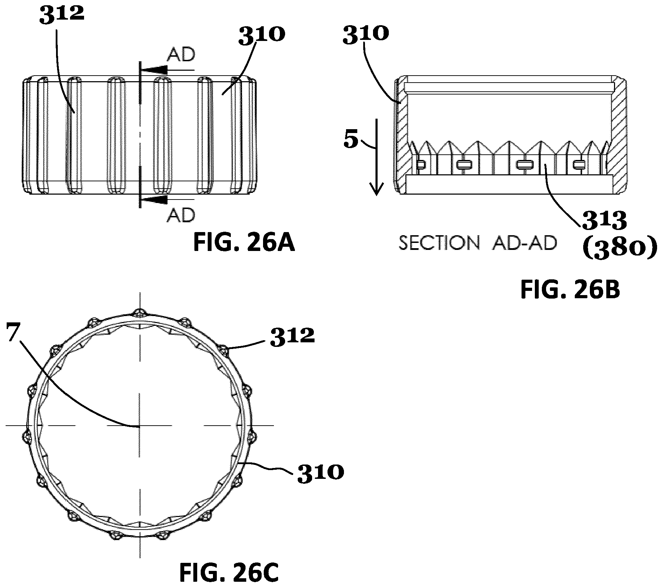

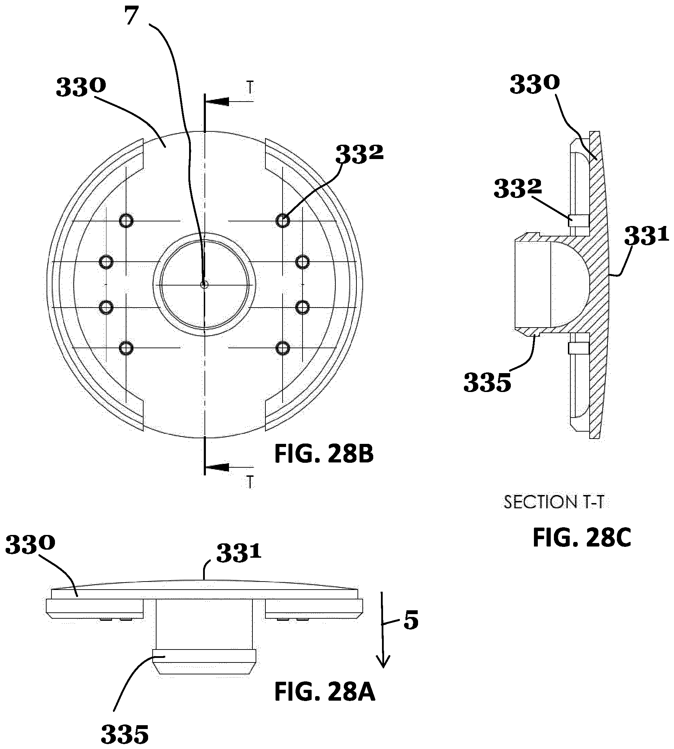

Figures 2A to 28C depict the abovementioned elements of the medicament delivery device 1 starting from the proximal end 2 in more detail.

Figs. 2A to 3C depict different views of the cap 10. The cap 10 has a generally cylindrical shape and defines an inner space 11 for receiving the container holder 40. The cap 10 is configured as a sleeve that is open at its distal end and closed at its proximal end. The cap 10 is configured as an injection molded part. It is made from plastic. The cap 11 comprises a protruding part 32 protruding from the generally cylindrical outer surface of the cap 10 that is configured to stop the cap 10 from rolling away on a plane surface. In other words, the protruding part 32 is configured to prevent rotation of the cap 10 and the medicament delivery mechanism 100 attached to the cap 10 when being placed on a flat surface. The protruding part 32 extends parallel to the longitudinal axis 7 and generally protrudes in the radial direction.

The cap 10 comprises an axial lock part 21 to releasably axially attach the cap 10 to the container holder 40. The axial lock part 21 may, for example, include a rib or a groove extending in a circumferential direction. The axial lock part 21 may be arranged on an inner circumferential surface of the cap 10. The cap 10 comprises a rotational lock part 22 to rotationally lock the cap 10 to the container holder 40 when the cap 10 is attached to the container holder 40. The rotational lock part 22 may, for example, include a rib or a groove extending in an axial direction. The rotational lock part 22 - in the shown embodiment - is configured as a pair of axially extending ribs on the inner circumferential surface of the cap 10 forming an indentation between the two ribs. The indentation is open along the longitudinal axis 7 in both the proximal direction 5 and the distal direction. The rotational lock part 22 may be arranged on an inner circumferential surface of the cap 10. In the shown embodiment, the axial lock part 21 and the rotational lock part 22 are arranged at a distal end section of the cap 10. The cap 10 may be formed by a single integral part. Alternatively, the cap 10 may be formed by two half shells. These half shells may be non-releasably connected to each other, e.g. glued together.

After attachment, the cap 10 covers the entire container holder 40 so that the container holder 40 does not form an outer surface of the medicament delivery device 1 when the cap 10 is attached.

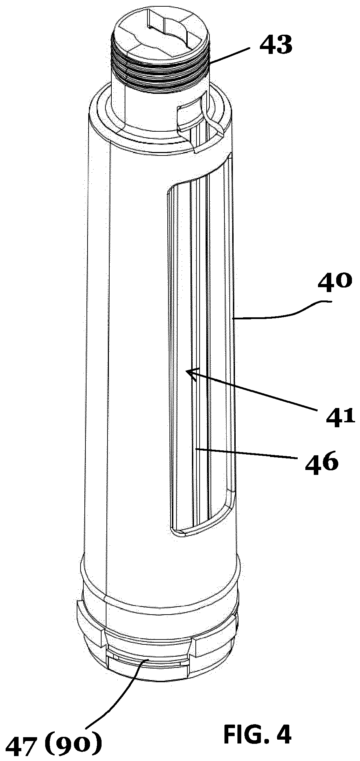

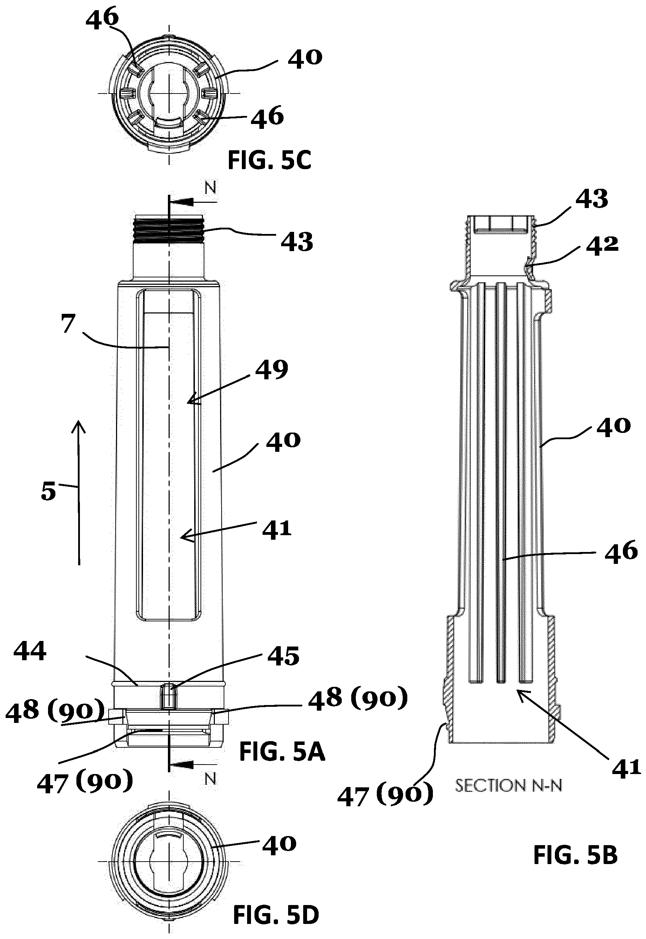

Figs. 4 to 5D depict different views of the container holder 40. The container holder 40 has a generally cylindrical shape and defines an inner space 41 for receiving the medicament container 500, such as a cartridge. The container holder 40 comprises a container connector 42 (cf. Fig. 5B) in form of a narrower section that allows to connect the medicament container 500 to the container holder 40 via a snap-fit engagement. Other connection concepts may be used instead of the snap-fit engagement.

The container holder 40 further comprises a needle connector 43 at its proximal end. The needle connector 43 is configured to secure a cannula to the container holder 40. Upon attachment of the cannula to the container holder 40, a fluid connection between the medicament container 500 and the cannula is established by piercing a septum provided at a proximal end of the medicament container 500. The needle connector 43 is exemplarily configured as a threaded connector. With other embodiments, the needle connector 43 may also be configured as a bayonet lock, a Luer lock or the like.

The container holder 40 further comprises an axial cap lock part 44 and a rotational cap lock part 45 (cf. Fig. 5A). The axial cap lock part 44 is configured to engage with the axial lock part 21 of the cap 10 to releasably hold the cap 10 at the container holder 40 in an axial direction. The axial cap lock part 44 is provided at the outer circumference of the container holder 40. Exemplarily, the axial cap lock part 44 is configured as a protrusion that protrudes from the outer surface of the container holder 40 and extends in the circumferential direction.

The rotational cap lock part 45 is configured to engage with the rotational lock part 22 of the cap 10 to block rotational movement of the cap 10 relative to the container holder 40 when the cap 10 is attached to the container holder 40. The rotational cap lock part 45 is provided at the outer circumference of the container holder 40. Exemplarily, the rotational cap lock part 45 is configured as a protrusion that protrudes radially from the outer surface of the container holder 40 and extends in the axial direction.

In the depicted embodiment, the cap 10 and the container holder 40 are attachable to each other in four different relative rotational orientations. Alternatively, rotational lock part 22 may be configured so that the cap 10 and the container holder 40 are attachable to each other in one, two, three or more than four different relative rotational orientations.

With the drug delivery device 1, the cap 10 is connected to the housing 160 of the medicament delivery mechanism 100 via the container holder 40. The cap 10 thereby only engages with the container holder 40 but not with the housing 160 or any other part of the medicament delivery mechanism 100.

As can be seen from Fig. 5A and Fig. 5B, the container holder 40 comprises multiple alignment parts 46 shown in the form of axially extending ridges, that are configured to align, in particular coaxially align, the medicament container 500 to the container holder 40.

The container holder 40 is configured to attach the medicament container 500 to the housing 160. In order to do so, the container holder 40 is non-releasably attached or attachable to a proximal end of the medicament delivery mechanism 100. The container holder 40 connects to the housing 160, namely to the body front part 180 of the housing 160, via a container connector 90. The connector 90 is configured as a snap fit connector. The connector 90 is configured to axially and rotationally fix the container holder 40 to the housing 160, namely to the body front part 180 of the housing 160.

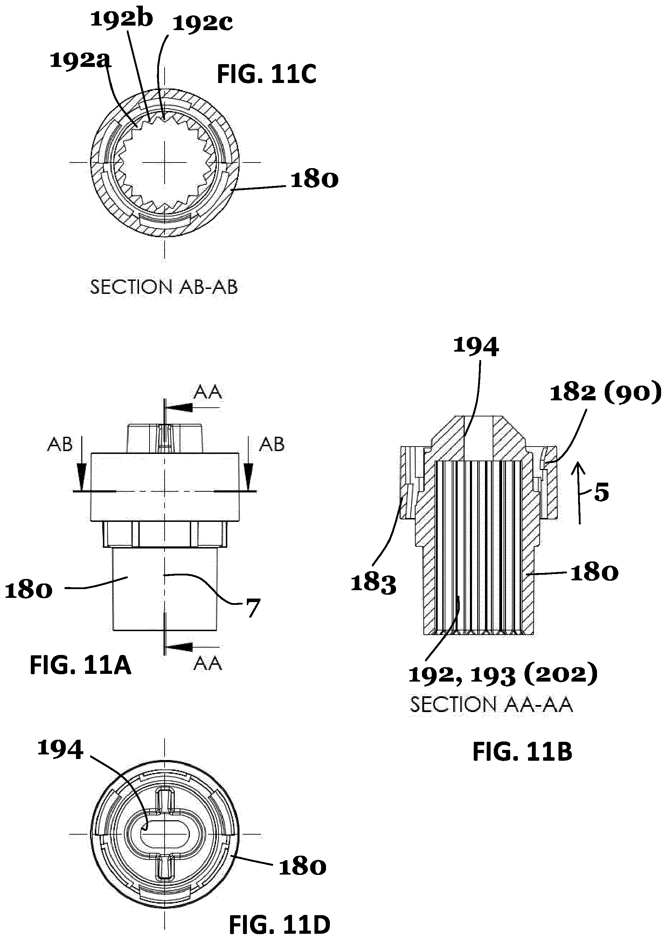

For axially connecting the container holder 40 to the housing 160, the container holder 40 comprises at least one coupling part 47, in the shown embodiment multiple coupling parts 47 arranged around the circumference of the container holder 40, that is configured to form a snap-fit connection with a corresponding coupling part 182 of the housing 160 (cf. Fig. 11B). In the shown embodiment, the coupling parts 47 of the container holder 40 each comprise a circumferentially extending tapering and the coupling parts 182 of the body front part 180 each comprise a flexible hook configured to engage with the tapering to form a snap fit connection. The coupling part 47 is arranged at a distal end portion of the container holder 40.

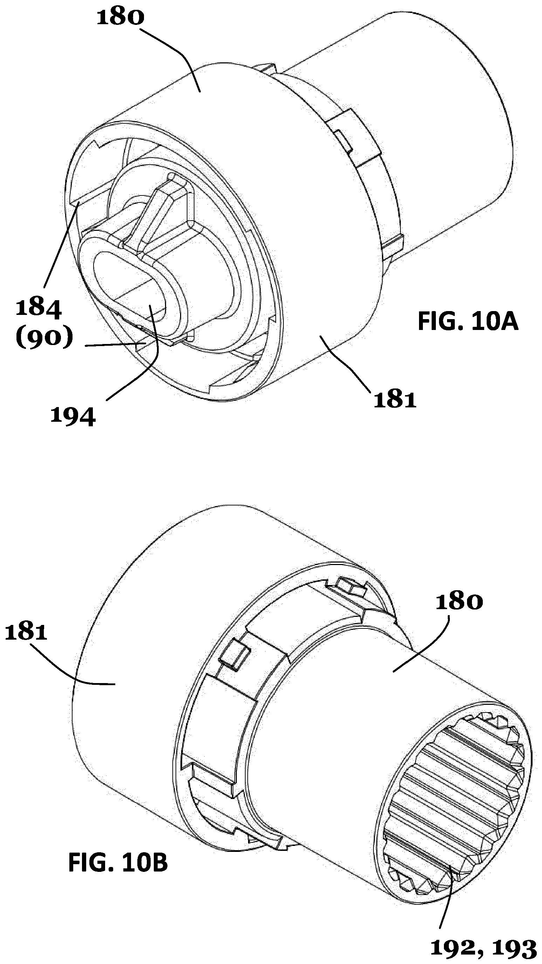

For rotationally connecting the container holder 40 to the housing 160, the container holder 40 comprises at least one rotational coupling part 48 (cf. Fig. 5A) that abuts a rotational coupling part 184 (cf. Fig. 10A) of the body front part 180. The rotational coupling part 48 of the container holder 40 and the rotational coupling part 184 of the body front part 180 cooperate with each other in an attached state to block rotational movement of the container holder 40 relative to the housing 160 in both rotational directions. The rotational coupling part 48 of the container holder 40 defines two surfaces facing each other and spaced apart in the circumferential direction. Between the two surfaces, the axial coupling part 47 is arranged. The rotational coupling part 184 of the body front part 180 defines two corresponding surfaces facing away from each other and spaced apart in the circumferential direction. Between the two corresponding surfaces, the axial coupling part 182 is arranged. The coupling part 48 is arranged at a distal end portion of the container holder 40.

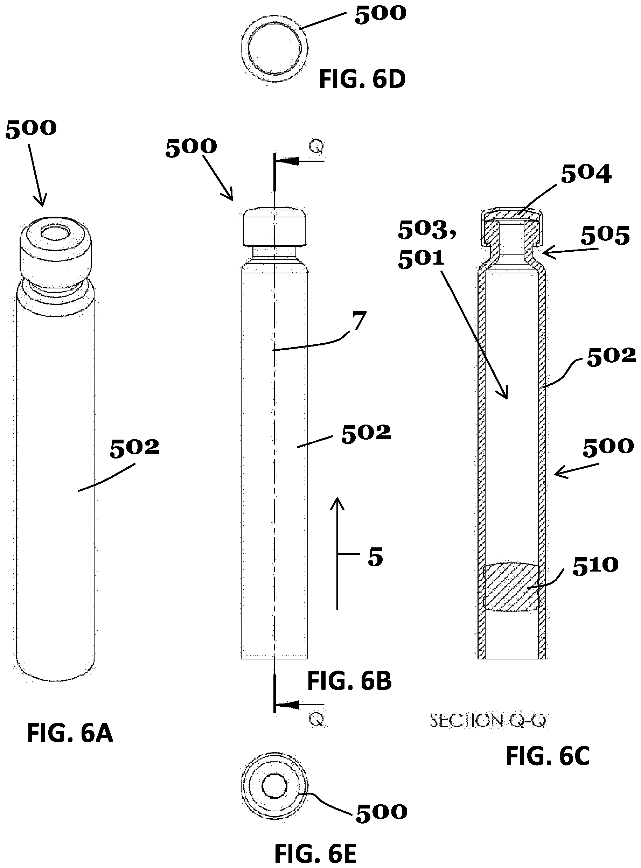

Figs. 6A to 6E show the medicament container 500. The medicament container 500 comprises a container body 502 that surrounds a medicament compartment 503 (cf. Fig. 6C) containing a medicament 501. A distal end of the medicament compartment 503 is sealed by a movable plunger 510 and a proximal end of the medicament compartment 503 is sealed by a septum 504. The plunger 510 and the septum 504 are made from an elastic material, such as a rubber. With alternative embodiments, the medicament compartment 503 may also be integrally formed with the container holder 40. The container holder 40 comprises two radially opposing windows 49 (cf. Fig. 5A) that allow for inspection of the medicament 501 within the container holder 40.

The medicament container 500 forms a connector 505. The connector 505 comprises a tapered section. The tapered section 505 is configured to form a snap-fit engagement with the container connector 42 of the container holder 40.

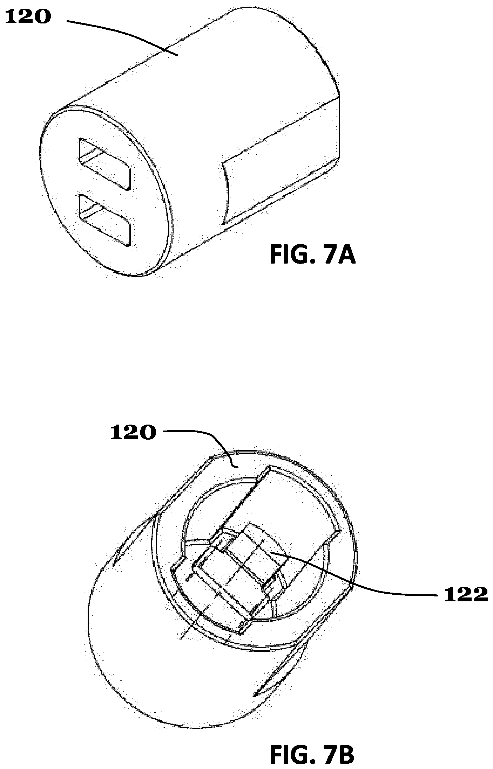

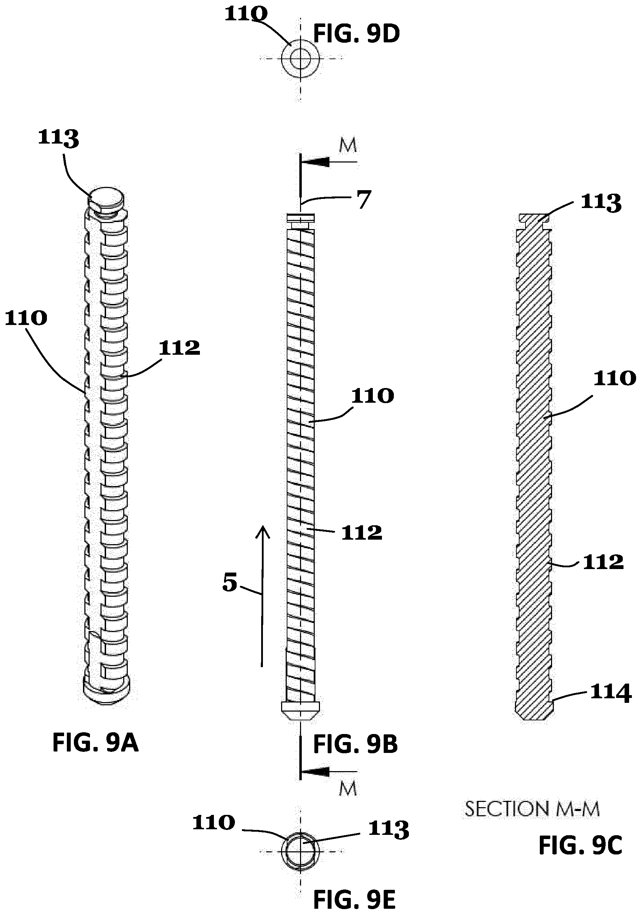

Figs. 7A to 8D depict the piston 120. The piston 120 is configured to exert a force onto the plunger 510 in the medicament container 500 to push the plunger 500 in the proximal direction 5. The piston 120 has a generally cylindrical shape. The piston 120 forms a passage opening extending from the proximal to the distal end of the piston 120 to simplify production of the piston 120 via injection molding. The piston 120 comprises a connector 122 to connect the piston 120 to the piston rod 110. The connector 122 is configured to connect the piston 120 to the piston rod 110 via a snap-fit connection. In particular, the connector 122 is configured to connect the piston 120 to the piston rod 110 by axially plugging the piston rod 110 into the piston 120. The connector 122 comprises multiple elastic bendable hooks that are configured to form a snap fit connection with a connecting portion 113 (cf. Fig. 9A) of the piston rod 110. The connecting portion 113 is arranged at a proximal end of the piston rod 110.

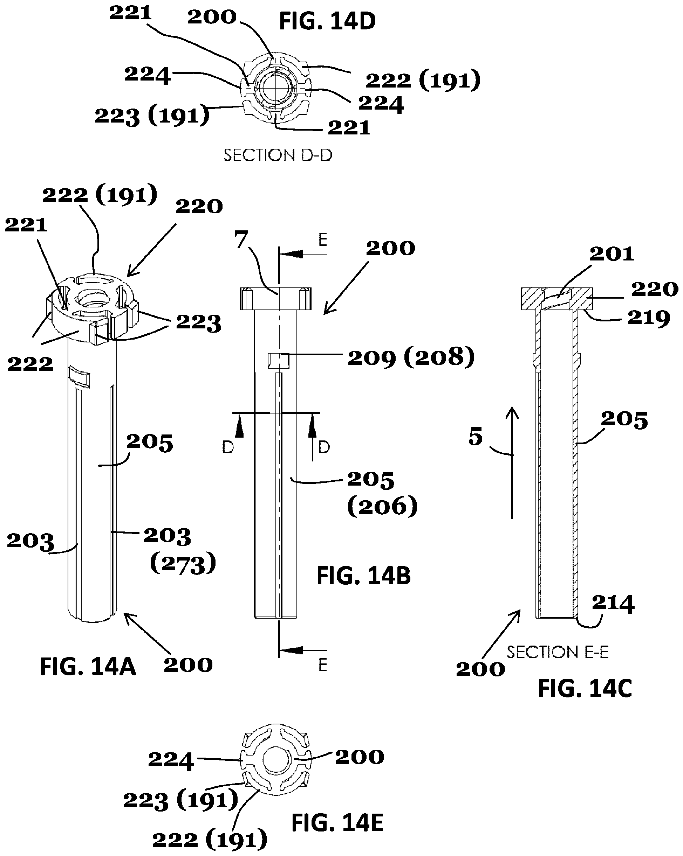

The piston rod 110 is depicted in Figs. 9A to 9E. During dose setting and dose delivery, the piston rod 110 is rotationally fixed with respect to the housing 160, in particular with the body front part 180. It is thereby axially guided within the housing 160, e.g. the body front part 180. As can be seen from Figs. 9A and 9B the piston rod 110 has a non-circular outer shape along a majority of its length. The outer shape is formed that way to allow the piston rod 110 to extend through an oval through hole forming a linear guide 194 (cf. Fig. 10A) for the piston rod 110 in the body front part 180 but to block relative rotational movement between the piston rod 110 and the body front part 180. The piston rod 110 comprises an outer thread 112 that meshes with an inner thread 201 (cf. Fig. 14C) of the nut 200. The piston rod 110 also forms a last dose stop 114 that prevents setting of a dose that is larger than a remaining amount of medicament in the medicament compartment 503. The last dose stop 114 is arranged at the distal end of the piston rod 110. The last dose stop 114 acts between the piston rod 110 and the nut 200. The last dose stop 114 comprises a radial protrusion on the outer surface of the piston rod 110, the radial protrusion being located at the distal end of the outer thread 112 of the piston rod 110. The last dose stop 114 is configured to engage with a distal end face 214 (cf. Fig. 14C) of the nut 200 when the nut 200 reaches the distal end of the piston rod 110 during dose setting. With each dose that is delivered, a zero dose position of the nut 200 with respect to the piston rod 110 sequentially moves in the proximal direction 5 along the piston rod 110. A length of the outer thread 112 on the piston rod 110 is adapted to cause engagement of the last dose stop 114 and to stop distal movement of the nut 200 when a set dose equals the remaining dose in the medicament compartment 503.

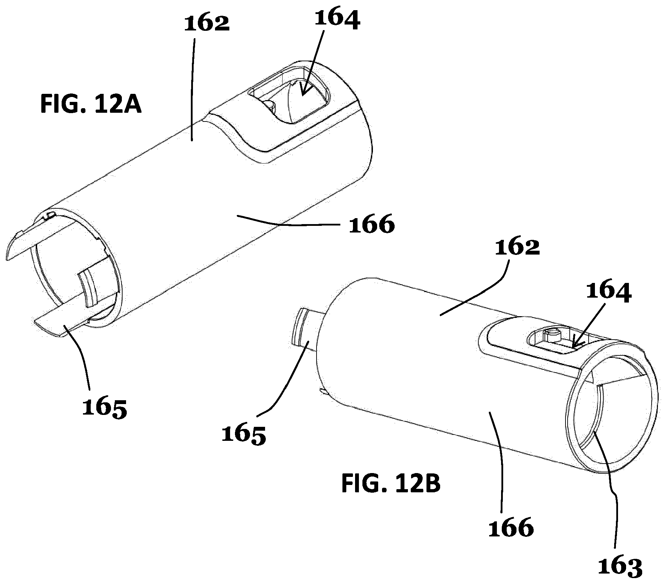

Figs. 10A to 11D depict the body front part 180. The body front part 180 is generally cylindrical and forms a through hole extending axially through the body front part 180. The body front part 180 is connectable or connected to the container holder 40 via the connector 90 so that the container holder 40 is positioned proximal to the body front part 180. The body front part 180 comprises a body connector 183 (cf. Fig. 11B) that is configured to non-releasably connect the body front part 180 to the body rear part 162 by engaging with the body connector 165 (cf. Fig. 12A). In the shown embodiment, the body front part 180 and the body rear part 162 are non-releasably attachable or attached via a snap fit connection. The body front part 180 and the body rear part 162 are attachable to each other by axially moving the body front part 180 and the body rear part 162 towards each other. Thereby, elastically bendable hooks of the body connector 165 engage with engagement surfaces of the body connector 183 to form the snap fit engagement. The body connector 183 and/or the body connector 165 may form insertion chamfers to decrease the force needed to attach the body front part 180 to the body rear part 162. In the shown embodiment, the body connector 183 is arranged at an inner circumferential surface of the body front part 180 and the body connector 165 is arranged at an outer circumferential surface of the body rear part 162.

The body front part 180 further comprises a linear guide 193 for the nut 200 and the drive sleeve 230. The linear guide 193 is created by a toothed inner circumferential surface 192. Each tooth 192a, 192b, 192c etc. of the toothed inner circumferential surface 192 acts as a dose stop 192a, 192b, 192c. Each dose stop 192a, 192b, 192c corresponds to a settable dose. The dose stops 192a, 192b, 192c each define a relative rotational position between the nut 200 and the body front part 180, i.e. the housing 160. The toothed inner circumferential surface 192 and the inner surface of the linear guide 194 for the piston rod 110 together define the through hole extending axially through the body front part 180. The coupling part 182 and the body connector 183 are not formed by the inner circumferential surface defining the through hole extending axially through the body front part 180. Instead, the coupling part 182 and the body connector 183 are arranged radially outwardly from the surface defining the through hole. The body front part 180 may be configured as an injection molded part.

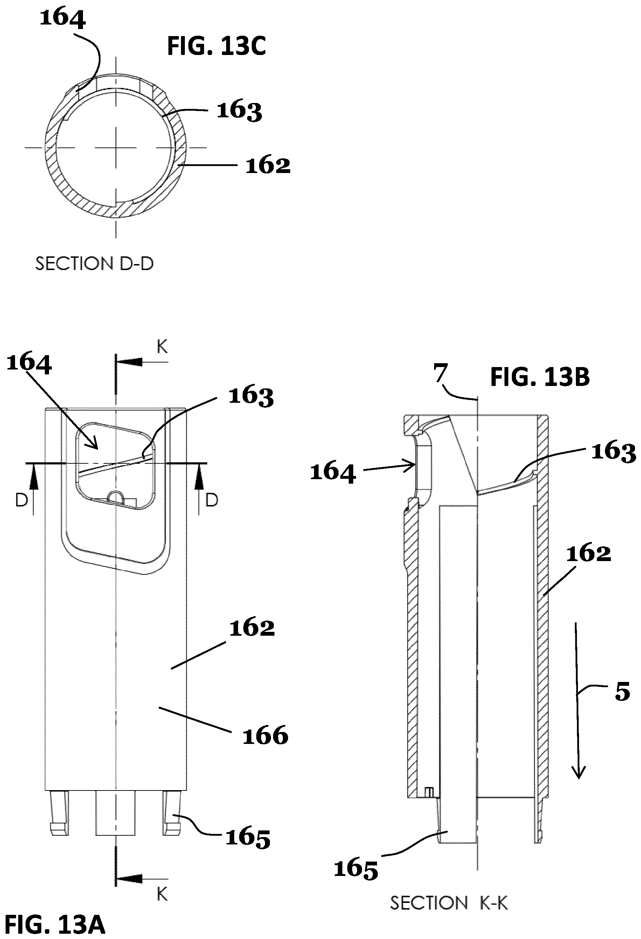

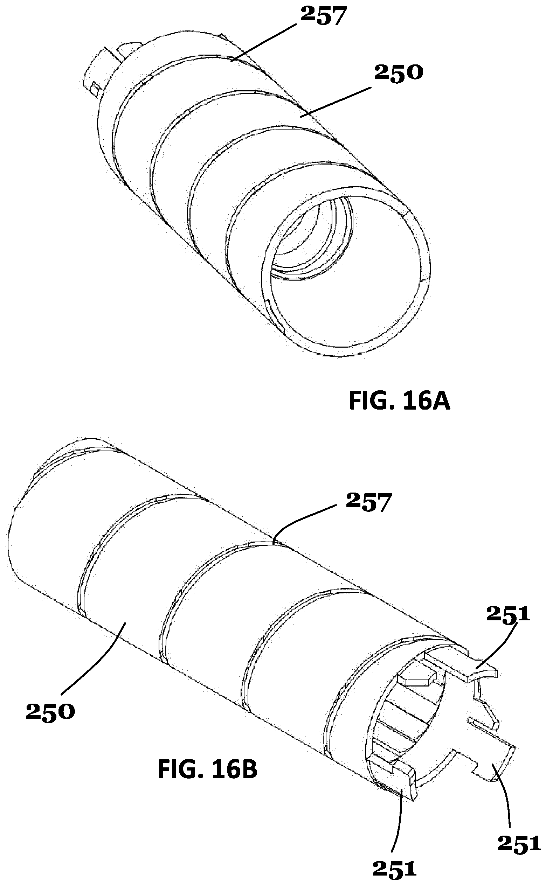

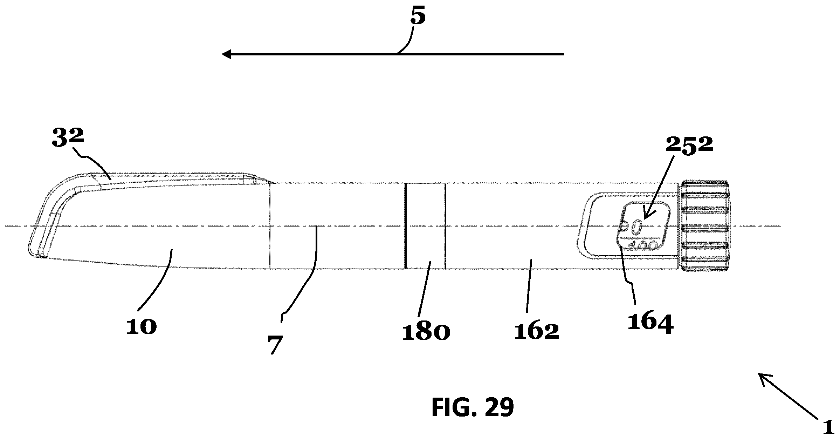

Figs. 12A to 13C depict the body rear part 162. The body rear part 162 has a generally cylindrical shape that extends parallel to the longitudinal axis 7. It is configured as a sleeve that is open at both longitudinal ends. At the proximal end of the body rear part 162 the body connector 165 is arranged. An outer surface 166 of the body rear part 162 acts as an outer surface of the medicament delivery mechanism 100 and the medicament delivery device 1. In the circumferential surface of the body rear part 162, a window or opening 164 is formed. A corresponding marking 252 (cf. Fig. 29) is shown in said window 164. As can be seen e.g. in Fig. 12B and 13B, the body rear part 162 comprises a thread 163 at an inner circumferential surface of the body rear part 162. The thread 163 is configured to mesh with an outer thread 257 (cf. Fig. 16B) of the dose setting sleeve 250. The body rear part 162 may be configured as an injection molded part.

Figs. 14A to 14E depict the nut 200. The nut 200 is configured as a generally cylindrical hollow member that at least partially surrounds the piston rod 110. The nut 200 comprises a generally cylindrical body 205 and a protruding part 220 that radially protrudes from the body 205. The protruding part 220 is exemplarily located at a proximal end of the body 205. Within a central bore of the protruding part 220, the nut 200 comprises the inner thread 201 meshing with the outer thread 112 of the piston rod 110.

The protruding part 220 comprises a latching part 223 of a latching mechanism 191 on its outer circumferential surface. The latching part 223 comprises a longitudinal protrusion located at a free end of a flexible arm 222. The flexible arm 222 extends in the circumferential direction and has a fixed end that is fixed to a bar 221 that radially extends from the body 205. The first latching part 223 is configured to flex in the radial direction perpendicular to the longitudinal axis 7. It furthermore is configured to elastically bend radially inward upon rotational movement of the nut 200 relative to the body front part 180 so that the latching part 223 is able to pass the teeth 192a, 192b, 192c of the toothed inner circumferential surface 192 of the body front part 180.

In total, the nut 200 comprises four of the latching parts 223. The latching parts 223 are located pairwise opposite each other with respect to the longitudinal axis 7. Furthermore, each latching part 223 is attached together with a neighboring latching part 223 to a common bar 221. The nut 200 thereby comprises two of the bars 221 located at opposite positions with respect to the longitudinal axis 7. In between two latching parts 223 that are connected to different ones of the bars 221, the nut 200 comprises two rigid arms 224 each of which is connected by an additional one of the bars 221 to the body 205.

Outer circumferential surfaces of the flexible arms 222 and outer circumferential surfaces of the rigid arms 224 are configured as cylindrical surfaces around the longitudinal axis 7. The circumferential surfaces of the flexible arms 222 and the circumferential surfaces of the rigid arms 225 thereby have the same radius.

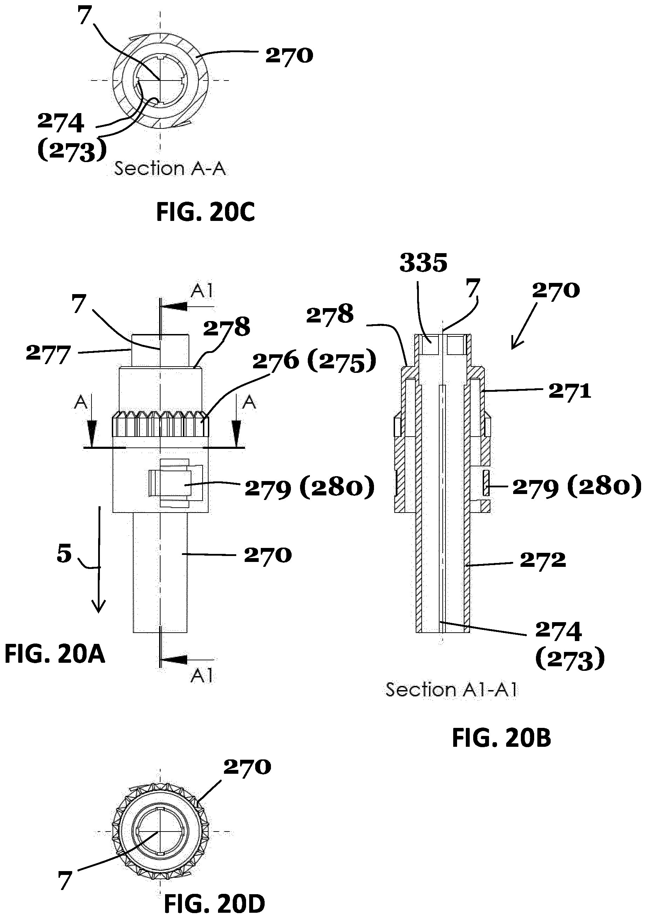

The nut 200 is rotationally fixed to the connector 270. For this purpose, the nut 200 comprises four longitudinal depressions 203 (cf. Fig. 14A) extending along the body 205 that engage with corresponding longitudinal ridges 274 (cf. Fig. 20C) on an inside surface of the connector 270. The longitudinal depressions 203 generally form first longitudinal elements of a rotation lock 273 that engage with corresponding second longitudinal elements of the rotation lock 273, which are formed by the longitudinal ridges 274.

The four longitudinal depressions 203 of the nut 200 are located pairwise opposite each other in the radial direction and are equally spaced from each other around the circumference of the nut 200. Each depression 203 is configured as a groove that extends in the axial direction with an open end at the distal end of the nut 200 so that the connector 270 can be pushed onto the nut 200 from the axial direction.

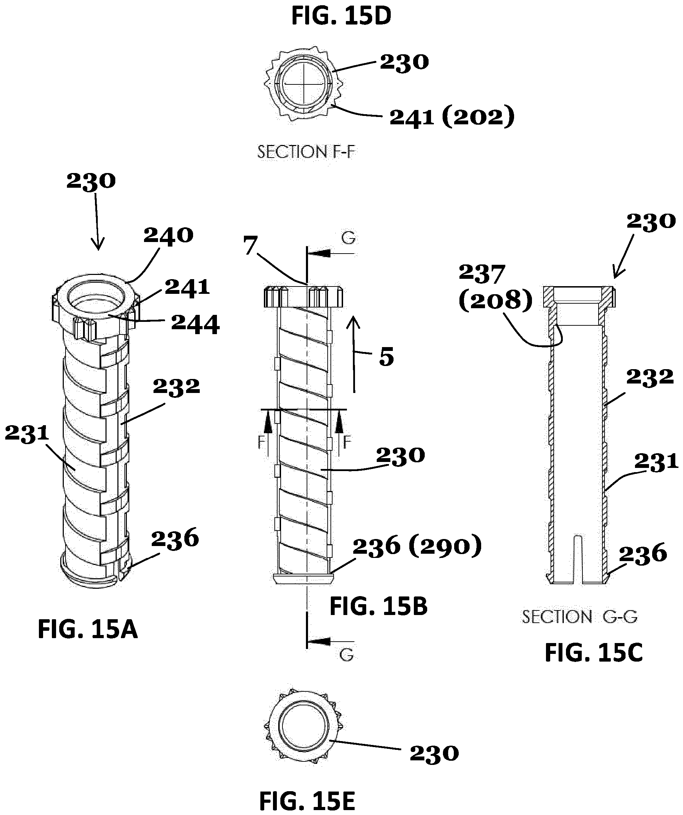

The nut 200 further comprises a first stop element 209 provided at an outer surface 206 of the nut 200. The stop element 209 engages with a second stop element 237 (cf. Fig. 15C) provided on an inner surface of the drive sleeve 230 to limit axial movement of the drive sleeve 230 in the distal direction with respect to the nut 200. Therefore, the first stop element 209 and the second stop element 237 act as a stop 208.

The first stop element 209 is configured as a radial protrusion on the outer surface 206 of the nut 200 and the second stop element 237 is configured as a radial protrusion on an inner surface of the drive sleeve 230. The first stop element 209 has an angled distal surface that is configured to allow relative movement of the drive sleeve 230 in the proximal direction 5 with respect to the nut 200 during assembly of the medicament delivery mechanism 100. A proximal surface of the first stop element 209 is configured as a radial surface perpendicular to the longitudinal axis 7. It prevents detachment of the drive sleeve 230 from the nut 200 after assembly.

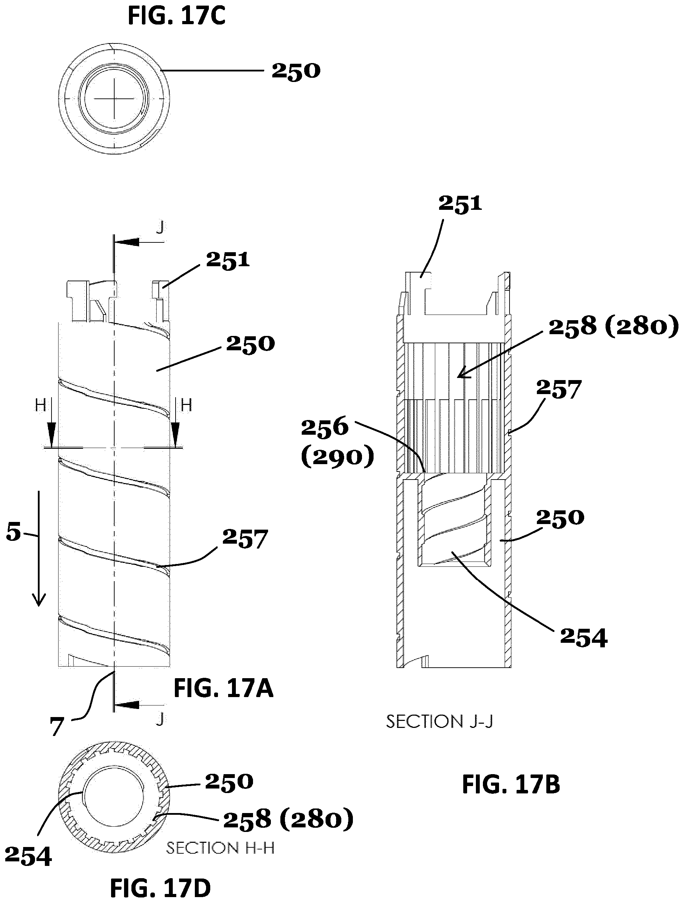

Figs. 15A to 15E depict the drive sleeve or driver 230. The drive sleeve 230 radially surrounds the body 205 of the nut 200. Furthermore, the drive sleeve 230 is located radially in between the nut 200 and the dose setting sleeve 250.The drive sleeve 230 comprises a body 232 that is configured as a hollow cylindrical member. The drive sleeve 230 comprises an outer thread 231 that is configured to mesh with an inner thread 254 of the dose setting sleeve 250. The outer thread 231 is provided on an outer circumferential surface of the body 232 of the drive sleeve 230. The outer thread 231 and the inner thread 254 are single-start threads. At the distal end of the body 232, the drive sleeve 230 comprises a radial protrusion. The radial protrusion comprises a first maximum dose stop surface 236 of a maximum dose stop 290. The first maximum dose stop surface 236 is configured to come in contact with a second maximum dose stop surface 256 (cf. Fig. 17B) when the medicament delivery mechanism 100 is set to a maximum settable dose. In Figs. 12A to 15D and 17B, the maximum dose stop 290 is configured as an axial dose stop.

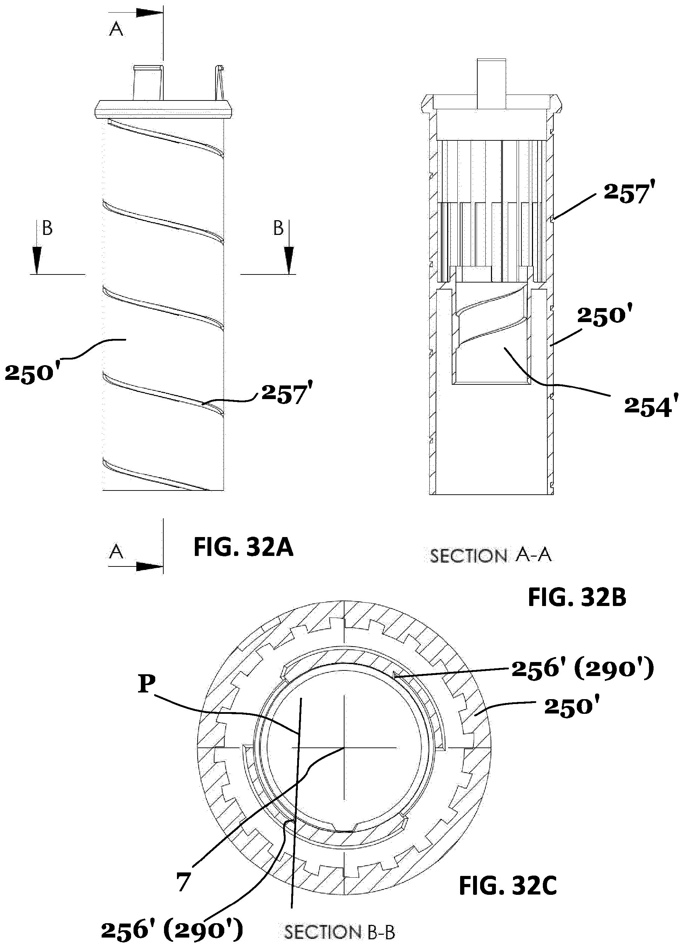

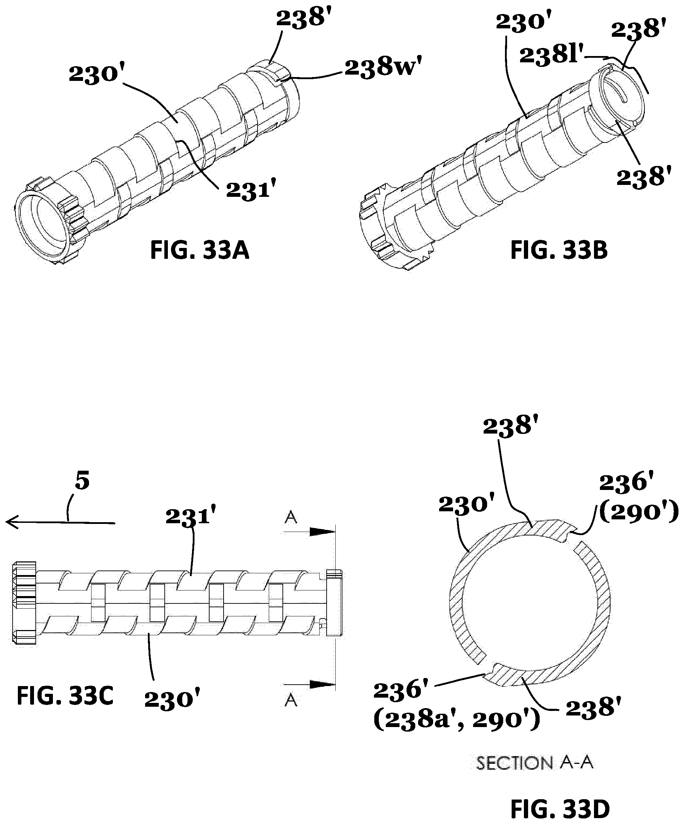

Figs. 32A to 34C depict an alternative embodiment, wherein the maximum dose stop 290' is configured as a radial dose stop. The maximum dose stop 290' is configured to act between the dose setting sleeve 250' (cf. Figs. 32A to 32C) and the drive sleeve 230' (cf. Figs. 33A to 33D).

The drive sleeve 230' is designed like the drive sleeve 230 besides its distal end portion. There, the drive sleeve 230' also comprises two elastically bendable portions 238' that are bendable in a radial direction. The two elastically bendable portions 238' extend along the circumferential direction of the drive sleeve 230'. The two elastically bendable portions 238' are located opposite each other in the radial direction and are equally spaced from each other around the circumference of the drive sleeve 230'. The two elastically bendable portions 238' are located at a distal end of the drive sleeve 230', i.e. they form the end of the drive sleeve 230'. However, the two elastically bendable portions 238' each have a free end with a front surface forming a radial front surface 238a' (cf. Fig. 33D). Each of the two radial front surfaces 238a' at the free end is configured as a first maximum dose stop surface 236'. The two elastically bendable portions 238' each have a constant width 238w' (cf. Fig. 33A) in the direction along the longitudinal axis 7. The two elastically bendable portions 238' each have a length 238I' (cf. Fig. 33B) measured along a circumferential direction 6. The length 238I' of each of the elastically bendable portions 238' is greater than the width 238w' of each of the two elastically bendable portions 238'.

The first maximum dose stop surfaces 236' are configured to come in contact with corresponding second maximum dose stop surfaces 256' (cf. Fig. 32C) of the dose setting sleeve 250' when the medicament delivery mechanism 100 is set to a maximum settable dose. The first maximum dose stop surface 236' (cf. Fig. 33D) and the second maximum dose stop surface 256' (cf. Fig. 32C) form oblique surfaces to force the elastically bendable portions 238' of the first member 230' towards the second member 250' when the first maximum dose stop surfaces 236' and the second maximum dose stop surfaces 256' come in contact with each other. "Oblique" means that the first maximum dose stop surfaces 236' and the second maximum dose stop surfaces 256' are positioned in a plane P (cf. Fig. 32C) that is spaced apart from the centerline or longitudinal axis 7 of the medicament delivery mechanism 100.

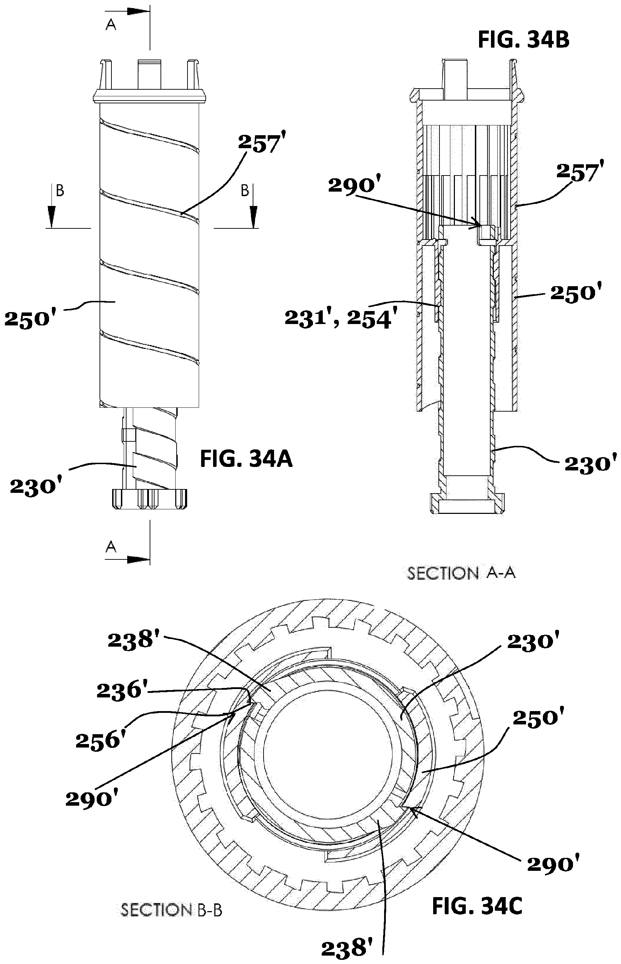

Figs. 34A to 34C show an assembly of the drive sleeve 230' and the dose setting sleeve 250' in a state when the medicament delivery mechanism 100 is set to a maximum settable dose, i.e. where the radial dose stop 290' is active. In Fig. 34C, it can be seen that, due to the oblique first maximum dose stop surface 236' and the oblique second maximum dose stop surface 256', the first maximum dose stop surface 236' and the second maximum dose stop surface 256' hook into each other. In other words, the first maximum dose stop surface 236' and the second maximum dose stop surface 256' interlock with each other. This creates a very reliable radial dose stop 290' since the oblique surfaces displace the elastically bendable portions 238' towards an inner circumferential surface of the dose setting sleeve 250' which avoids that the elastically bendable portions 238' are displaced inwardly and therefore avoids that the first maximum dose stop surface 236' passes the second maximum dose stop surface 256' if a user tries to set the medicament delivery mechanism 100 to a dose higher than the maximum settable dose.

The drive sleeve 230/230' of both embodiments further comprises a protruding part 240 (cf. Fig. 15A) that radially protrudes from the body 232. The protruding part 240 is located at a proximal end of the drive sleeve 230.

At its outer circumferential surface, the protruding part 240 comprises a lock element 241 of a rotation lock 202 between the drive sleeve 230 and the housing 160, i.e. the body front part 180. The first lock element 241 is configured as a radial tooth extending radially from the outer surface of the drive sleeve 230 and being elongated along the longitudinal axis 7. As can be seen from Figs. 15A and 15D, the drive sleeve 230 comprises several of the first lock elements 241 that are distributed around the circumference of the drive sleeve 230.

The drive sleeve 230 is configured to push the nut 200 in the proximal direction during dose delivery. To do so, the drive sleeve 230 comprises a contact surface 244 in form of an end face at the protruding part 240 and the nut 200 forms a corresponding second contact surface 219 (cf. Fig. 14C) that engage with each other during dose delivery. The second contact surface 219 is provided at the protruding part 220 of the nut 200. The second contact surface 219 is formed by a distal facing surface of the nut 200. The distal facing surface thereby forms a distal facing end surface of the protruding part 220.

Figs. 16A to 17D depict the dose setting sleeve 250 shown in Fig. 1. The dose setting sleeve 250 and the alternative dose setting sleeve 250' generally are designed the same way except for the section forming the maximum dose stop. The dose setting sleeve 250 comprises a connector 251 at its distal end. The connector 251 is configured to axially and rotationally fix the dose setting sleeve 250 to the coupling element 370. The connector 251 comprises multiple hooks that extend in the axial direction. The connector 251 is configured as a snap-fit connector that allows to snap the coupling element 370 onto the dose setting sleeve 250 from the distal direction during assembly.

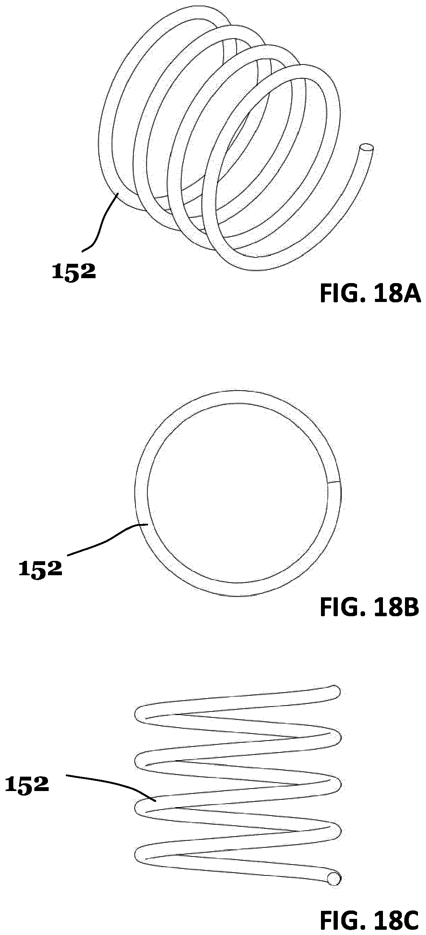

As can be seen from Fig. 17B, the inner thread 254 of the threaded connection between the drive sleeve 230 and the dose setting sleeve 250 is provided on an inside surface of an inner sleeve of the dose setting sleeve 250. The inner sleeve is connected to an outer tubular portion of the dose setting sleeve 250 by a shoulder. The shoulder provides a bearing surface for the biasing member 152 (cf. Figs. 18A to 18C). The inner sleeve provides a centering function for the biasing member 152. The inner thread 254 of the threaded connection extends over the entire length of the inner tubular portion. A distal end face of the inner tubular portion provides the second maximum dose stop surface 256 of the maximum dose stop 290.

The dose setting sleeve 250 comprises the markings 252 (cf. Fig. 29) on its outer surface that are visible through the window 164 in the body rear part 162 and that indicate the set dose. The markings 252 (not shown in Figs. 16A to 17D) are arranged in a helical pattern (cf. Fig. 1) on an outer surface of the dose setting sleeve 250. The dose setting sleeve 250 forms a dose indication member of the medicament delivery mechanism 100.

At the outer circumferential surface, the dose setting sleeve 250 comprises the outer thread 257. The outer thread 257 meshes with the inner thread 163 at the inner circumferential surface of the body rear part 162.

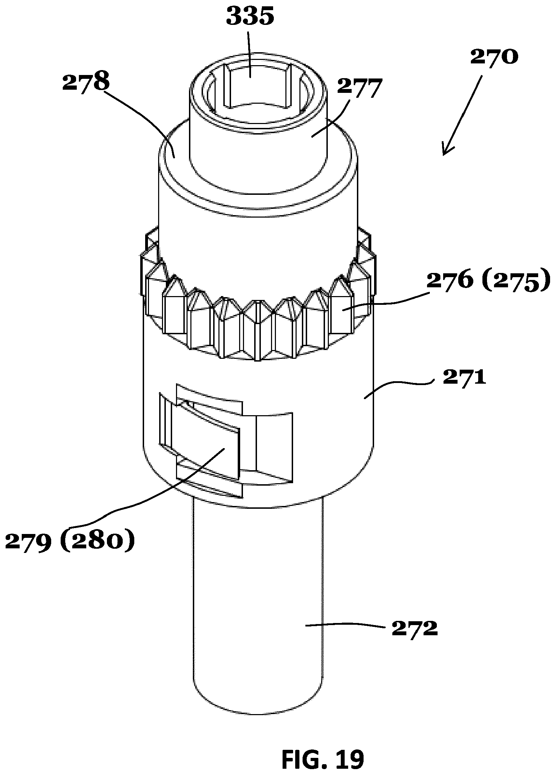

Distally from the inner sleeve, the dose setting sleeve 250 comprises a toothed inner circumferential surface 258 that engages with a flexible part 279 of the connector or coupling member 270 to act as a feedback mechanism 280. The feedback mechanism 280 provides an audible and/or tactile feedback to a user during dose delivery. The feedback mechanism 280 more precisely comprises two flexible parts 279 that protrude radially from an outer surface of a clutch part 271 (cf. Fig. 19) of the connector 270. The flexible parts 279 are located opposite each other in the radial direction.

The flexible parts 279 of the feedback mechanism 280 engage with radial teeth of the toothed inner surface 258 of the dose setting sleeve 250. The feedback mechanism 280 is active only during dose delivery but not during dose setting since the connector 270 and the dose setting sleeve 250 are rotationally fixed with each other during dose setting.

Figs. 19 to 20D depict the connector 270. As can be best seen from Fig. 20B, the connector 270 has an inner section that can be defined as the connection part 272 and an outer section that can be defined as the clutch part 271. The connection part 272 forms a tubular portion having a through hole. At an inner circumferential surface of the connection part 272, the ridges 274 of the rotation lock 273 are arranged. The ridges 274 extend along the axial direction and over a majority of a length of the connector 270. The connection part 272 is surrounded by a tubular clutch part 271 that comprises first clutch parts 276 of a clutch or clutch mechanism 275 on its outer surface. The first clutch parts 276 are configured as radially extending teeth.

The clutch part 271 is joined to the connection part 272 at the distal end of the clutch part 271, where it forms the shoulder 278 that is in axial engagement with the bearing 360 via the proximal of the two washers 261 in the dose setting state. A distal cylindrical part 277 extends distally from the shoulder 278 and has a reduced diameter compared to the clutch part 271. On the inside surface of the distal cylindrical part 277, an axial lock 335 is formed. The axial lock 335 is configured to axially fix the connector 270 to the button 330.

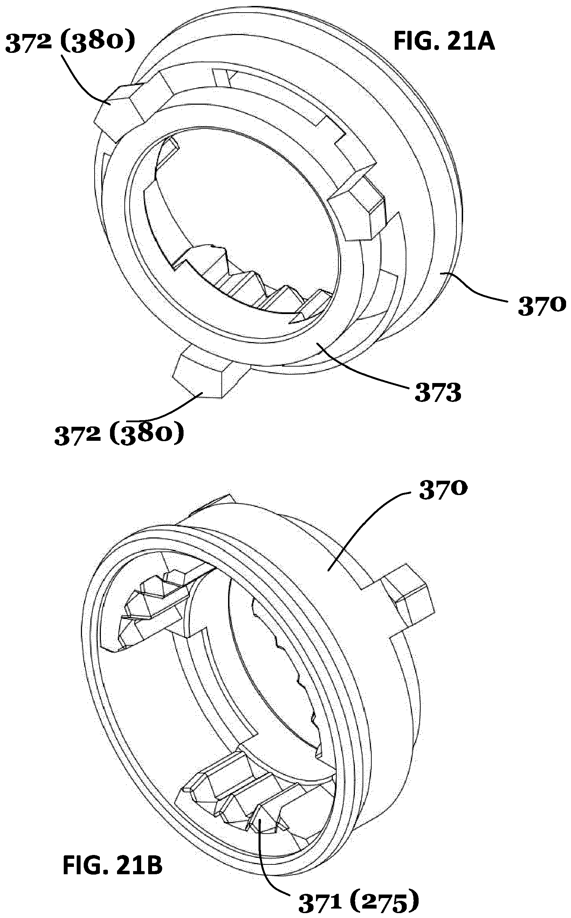

Figs. 21A to 22D depict the coupling member 370. The coupling member 370 and the connector 270 together act as the clutch or clutch mechanism 275. The coupling member 370 is generally cylindrically shaped. The coupling member 370 comprises second clutch parts 371 that are configured to engage with the first clutch parts 276 to rotationally fix the coupling member 370 and the connector 270. The second clutch parts 371 are configured as radially extending teeth. The second clutch parts 371 are arranged at a proximal section of the coupling element 370.

More precisely, the clutch mechanism 275 comprises radial teeth 276 on an outer surface of the connector 270 that engage with corresponding radial teeth 371 on an inner surface of the coupling element 370 when the clutch 275 is in the closed state. The clutch 275 is configured to transfer from the closed state into the opened state by axially moving the connector 270 with respect to the coupling element 370. During dose setting, the clutch 275 is biased into its closed state. The biasing member 152 is provided between the connector 270 and the dose setting sleeve 250 and biases the connector 270 in the distal direction with respect to the dose setting sleeve 250. As can be seen from Figs. 18A to 18C, the biasing element 152 is configured as a compression spring.

The clutch mechanism 275 generally acts between the nut 200 and the dose setting sleeve 250. The clutch 275 rotationally locks the nut 200 to the dose setting sleeve 250 in a closed state, i.e. when the first clutch parts 276 and the second clutch parts 371 are brought into meshing engagement, and allows relative rotation between the nut 200 and the dose setting sleeve 250 in an opened state, i.e. when the first clutch parts 276 and the second clutch parts 371 are not in meshing engagement. During dose setting, the clutch mechanism 275 rotationally locks the nut 200 to the dose setting element 250 and, during dose delivery, the clutch mechanism 275 rotationally decouples the nut 200 from the dose setting element 250. The clutch mechanism 275 can be decoupled by pushing the button 330 in the proximal direction 5. Proximal movement of the button 330 moves the connector 270 in the proximal direction 5 against the force of the biasing member 152. This transfers the clutch mechanism 275 from its closed state into its opened state.

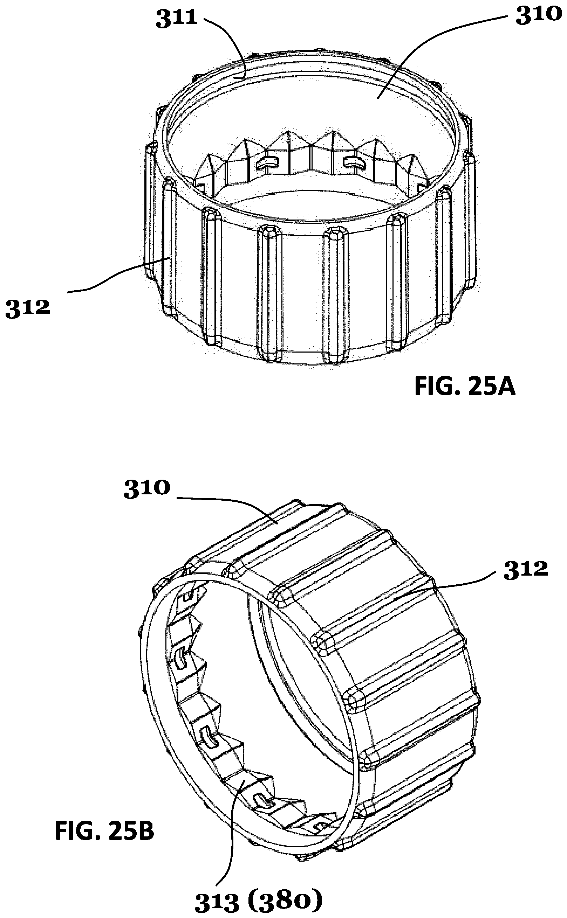

The coupling element 370 and the dose setting element 310 (cf. Figs. 25A to 26C) form a second clutch mechanism 380. The coupling element 370 forms first coupling sections 372 that are configured to engage with second coupling sections 313 of the dose setting element 310 to rotationally fix the coupling element 370 and the dose setting element 310. The first coupling sections 372 are arranged at an outer circumference of the coupling element 370. The first coupling sections 372 are configured as radially extending teeth. The first coupling sections 372 are arranged at a distal section of the coupling element 370.

The second coupling sections 313 of the second clutch mechanism 380 are formed by the dose setting element 310. The second coupling sections 313 are arranged at an inner circumference of the dose setting element 310. The second coupling sections 313 are configured as radially extending teeth. The second coupling sections 313 are arranged at a proximal section of the dose setting element 310. The coupling element 370 is arranged at least partly inside the dose setting element 310.

The second clutch mechanism 380 is configured to rotationally fix the coupling element 370 and thereby the dose setting sleeve 250 to the dose setting element 310 in a closed state. Furthermore, the second clutch mechanism 380 is configured to allow relative rotational movement between the coupling element 370 and thereby the dose setting sleeve 250 and the dose setting element 310 in an open state. The second clutch mechanism 380 is brought into an open state by the user by pushing the button 330 into the proximal direction 5. The second clutch mechanism 380 - in the open state, i.e. during dose delivery - allows rotational movement of the dose setting sleeve 250, e.g. back to a zero-dose position, while making sure that the dose setting element 310 does not rotate during dose delivery.

As can be seen e.g. from Figs. 25A and 25B, the dose setting element 310 is generally cylindrically shaped. The dose setting element 310 has an outer circumferential surface with a gripping structure 312. The dose setting element 310 is configured to be gripped by a user at the gripping structure 312 and to be rotated by the user around the longitudinal axis 7 of the medicament delivery device 1. The gripping structure 312 simplifies rotating the dose setting element 310. More precisely, the gripping structure 312 is longitudinally structured to provide an enhanced grip to a user of the device. Upon setting a dose, a corresponding marking 252 is shown in the window 164 in the body rear part 162 of the medicament delivery mechanism 100. To reduce a set dose, the user rotates the dose setting element 310 back towards its zero position.

The dose setting element 310 has a central through hole that receives the distal cylindrical part 277 of the connector 270. At its distal end, the dose setting element 310 forms a circumferentially extending groove 311 that receives the button 330. The button 330 is axially connected to the dose setting element 310 via a snap-fit engagement by pushing the button 330 into the groove 311. The button 330 is rotatable relative to the dose setting element 310.

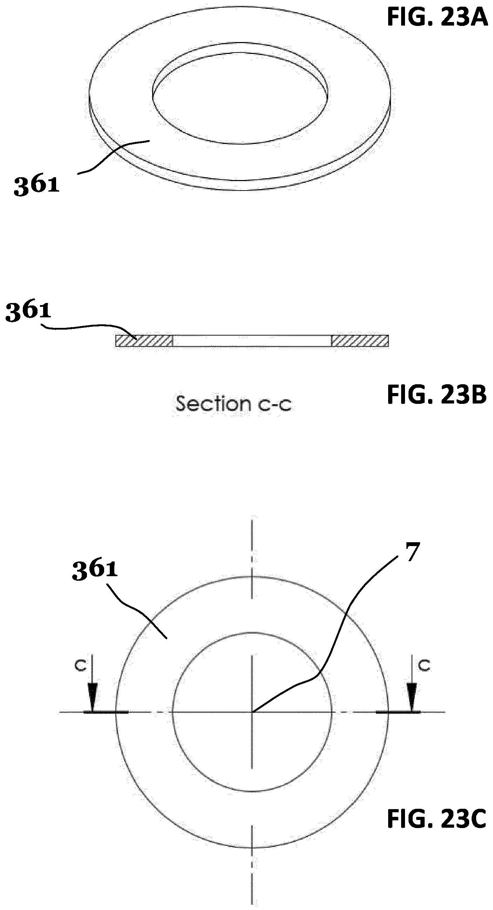

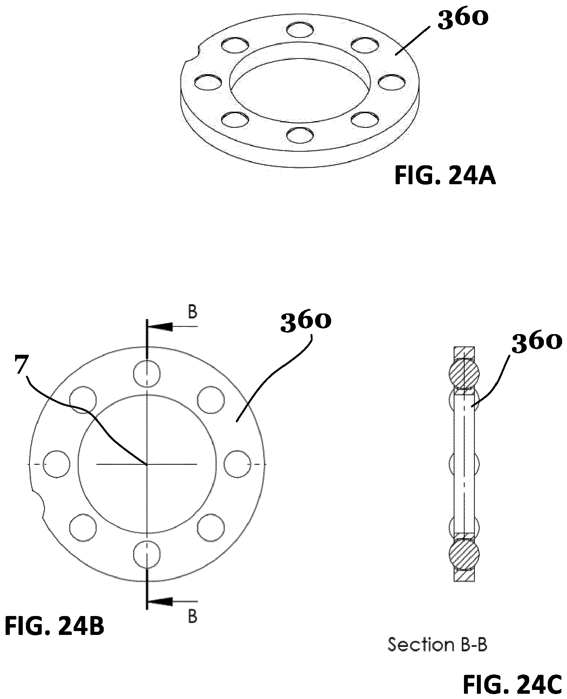

Figs. 24A to 24C depict the bearing 360. The bearing 360 is located between the button 330 and coupling element 370, i.e. between a proximal portion of the button 330 and a distal end face of the coupling element. The bearing 360 is configured as a thrust bearing with two opposing bearing surfaces that are each in contact with one of the washers 361 (Fig. 23A to 23C). The washers 361 are orientated perpendicular to the longitudinal axis 7. The bearing 360 exemplarily comprises a ball bearing. The bearing 360 is configured to facilitate rotation of the coupling element 370 and the dose setting sleeve 250 with respect to the button 330. Furthermore, the bearing 360 is configured to transfer an axial force from the button 330 to the coupling element 370 when the button 330 is pushed in the proximal direction to eventually apply an axial force onto the dose setting sleeve 250 to make the dose setting sleeve 250 rotate back to its original position, e.g. zero-dose position. The bearing 360 acts between the two washers 361. The proximal washer 361 is arranged between the bearing 360 and a contact surface 373 (cf. Fig. 21A) of the coupling element 370. The distal washer 361 is arranged between the bearing 360 and projections 332 arranged on the proximal side of the button 330.

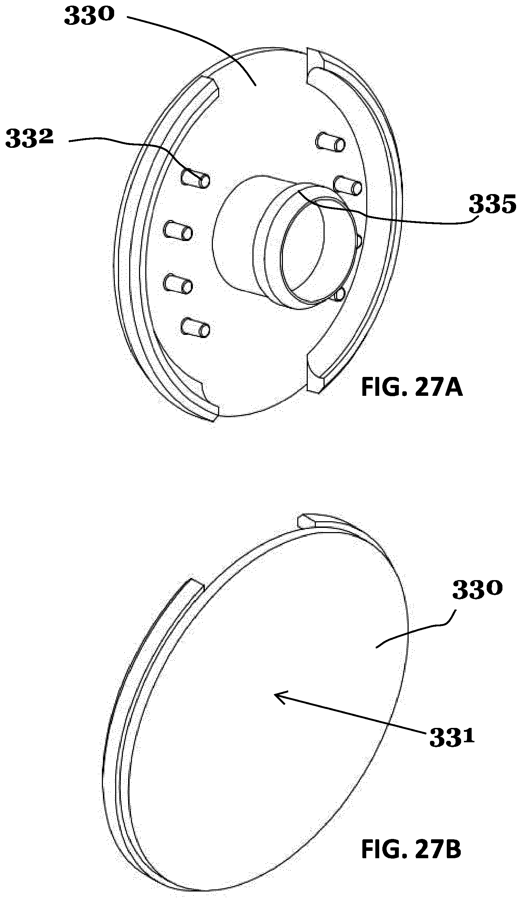

Figs. 27A to 28C depict the button 330. The button 330 is generally disc shaped. A distal side of the button 330 functions as a push surface 331. A user pushes onto the push surface 331 during dose delivery. At a proximal side of the button 330, the button 330 comprises a tubular portion forming the axial lock 335. The tubular portion has radially extending ridges on its outer circumferential surface which engage with corresponding radial protrusions on an inside surface of the distal cylindrical part 277 of the connector 270. The tubular portion of the button 330 is thereby received within the distal cylindrical part 277. Around the tubular portion of the button 330, the multiple projections 332 are formed that are in contact with the distal washer 361.

Fig. 29 depicts the medicament delivery device 1 in an assembled state. The cap 10 covers the container holder 40 completely so that the container holder 40 is not visible when the cap 10 is attached to the rest of the medicament delivery device 1. Next to the cap 10 in the distal direction, the body front part 180 is visible. Next to the body front part 180, the body rear part 162 is visible. The body rear part 162 forms the window 164 through which the user can see markings 252 that tell the user the dose that is currently set. Fig. 29 therefore shows the medicament delivery device 1 in a zero-dose state.

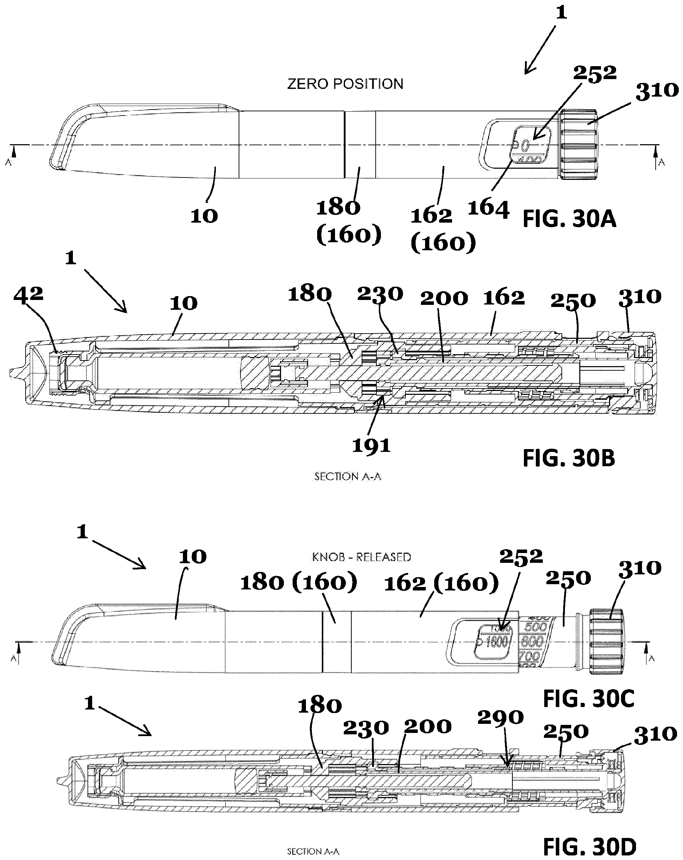

Figs. 30A and 30B also depict the medicament delivery device 1 in a zero-dose state or zero position. Usually, the medicament delivery device 1 is initially provided to the user in this zero-dose state. Contrary to the figures shown, the cap 10 is usually removed and a needle is connected to the needle connector 42 before the user starts setting a dose.

During dose setting, the user turns the dose setting element 310 relative to the housing 160. Thereby, as can be seen from comparing Figs. 30A and 30B with Figs. 30C and 30D, the nut 200, the dose setting element 310 and the dose setting sleeve 250 move in the distal direction relative to the housing 160. This is because both the nut 200 and the dose setting sleeve 250 are rotationally fixed to the dose setting element 310. Rotation of the dose setting element 310 therefore causes the nut 200 and the dose setting sleeve 250 to rotate and to thereby move axially with respect to the piston rod 110 and the housing 160 due to, on the one hand, the threaded connection between the outer thread 112 of the piston rod 110 and the inner thread 201 of the nut 200 and, on the other hand, the threaded connection between the outer thread 257 of the dose setting element 250 and the inner thread 163 of the body rear part 162 of the housing 160. Upon increasing a set dose, the nut 200 and the dose setting element 250 both move axially further in the distal direction. A set dose may be decreased by rotating the dose setting element 310 in an opposite direction as during dose setting. When the set dose is decreased, the nut 200 and the dose setting element 250 move axially in the proximal direction.

A pitch of the threaded connection between the outer thread 112 of the piston rod 110 and the inner thread 201 of the nut 200 is different from a pitch of the threaded connection between the outer thread 257 of the dose setting element 250 and the inner thread 163 of the body rear part 162 of the housing 160 so that the nut 200 and the dose setting element 250 travel differing axial distances upon rotation by the same angle. With the drug delivery device 1, the pitch of the threaded connection between the outer thread 112 of the piston rod 110 and the inner thread 201 of the nut 200 is smaller than the pitch of the threaded connection between the outer thread 257 of the dose setting element 250 and the inner thread 163 of the body rear part 162 of the housing 160 so that the dose setting element 250 travels a larger axial distance than the nut 200.

A pitch of the threaded connection acting between the rotationally fixed drive sleeve 230 and the dose setting sleeve 250, namely the outer thread 231 of the drive sleeve 230 and the inner thread 254 of the dose setting sleeve 250 is adapted to cause the drive sleeve 230 to axially move together with the nut 200 along the longitudinal axis 7 upon rotation of the dose setting element 310 during dose setting. The pitch of the outer thread 231 of the drive sleeve 230 and the inner thread 254 of the dose setting sleeve 250 thereby is at most a difference between a maximum pitch of the threaded connection between the outer thread 112 of the piston rod 110 and the inner thread 201 of the nut 200 and a minimum pitch of the threaded connection between the outer thread 257 of the dose setting element 250 and the inner thread 163 of the body rear part 162 of the housing 160. The maximum pitch of the threaded connection between the outer thread 112 of the piston rod 110 and the inner thread 201 of the nut 200 thereby is given by an upper limit of fabrication tolerances of the threaded connection between the outer thread 112 of the piston rod 110 and the inner thread 201 of the nut 200. The minimum pitch of the threaded connection between the outer thread 257 of the dose setting element 250 and the inner thread 163 of the body rear part 162 of the housing 160 is given by a lower limit of fabrication tolerances of the threaded connection between the outer thread 257 of the dose setting element 250 and the inner thread 163 of the body rear part 162 of the housing 160. This prevents the nut 200 from advancing faster in the distal direction than the drive sleeve 230 and avoids blocking movement of the nut 200 by the drive sleeve 230.

Furthermore, during dose setting, the latching mechanism 191 acts between the nut 200 and the body rear part 162. The nut 200 thereby rotationally couples the dose setting element 310 and the dose setting sleeve 250 to the latching mechanism 191. The latching mechanism 191 also acts as a dose definition mechanism that defines discrete rotational positions of the nut 200 and the dose setting element 310 and the dose setting sleeve 250, wherein the discrete rotational positions correspond to settable doses.

Relative rotation of the nut 200 with respect to the housing 160 during dose setting causes the latching part 223 to rotate with respect to the toothed inner circumferential surface 192 and to thereby provide an audible and/or tactile feedback to the user. The feedback comprises a click each time a dose has been set. The feedback is produced by the latching part 223 engaging with constitutive grooves in between the teeth 192a, 192b, 192c of the toothed inner circumferential surface 192.

Fig. 30D depicts a cross-sectional side view of the medicament delivery mechanism 100 after having set a maximum dose. The dose setting sleeve 250 has been screwed out of the housing 160 in the distal direction and the dose setting element 310 and the button 330 have moved together with the dose setting element 250 in the distal direction. The nut 200 has been screwed along the piston rod 110 in the distal direction and the drive sleeve 230 has translated together with the nut 200 in the distal direction without rotation. To set the maximum dose, the nut 200 and the dose member 250 have performed at least one, namely at least 1.5 revolutions around the longitudinal axis 7. Exemplarily, the maximum dose is set by rotating the nut 200 and the dose member 250 by more than 1.5 but less than two revolutions around the longitudinal axis 7.

The end stop 290 (cf. Fig. 30D) prevents setting of a dose that exceeds the maximum dose. The end stop 290 acts between the dose setting sleeve 250 and the drive sleeve 230. The end stop 290 limits axial movement of the drive sleeve 230 with respect to the dose setting sleeve 250. In Fig. 30D, the end stop 290 is configured as an axial stop so that stop surfaces 236, 256 of the end stop 290 engage by axially moving towards each other. However, as can be seen in Figs. 33A to 34C, alternatively, the end stop 290' may be configured as a radial stop so that stop surfaces 236', 256' of the end stop 290' engage moving towards each other along a circumferential direction. The end stop 290/290' is located at a distal end of the outer thread 231 of the drive sleeve 230.

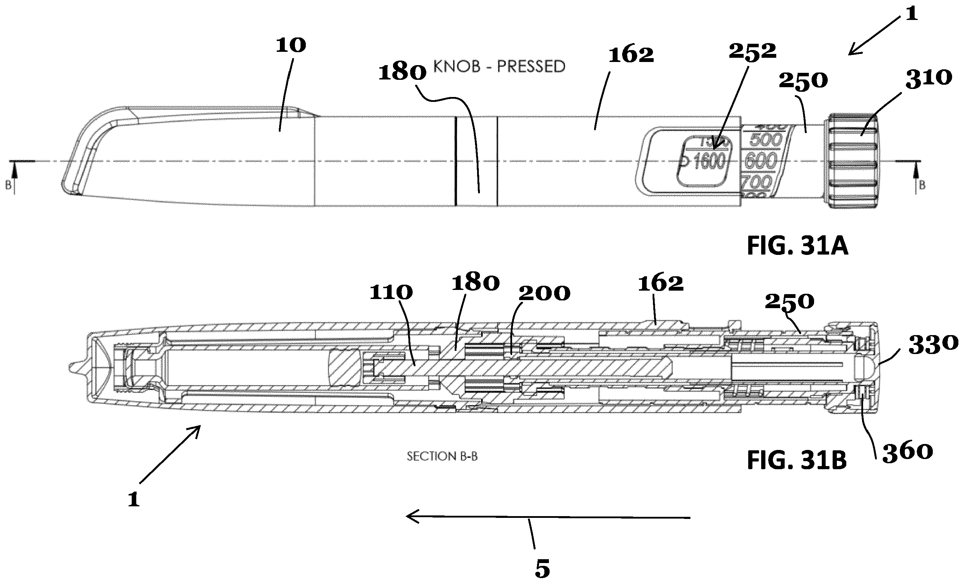

After having set the dose, the medicament delivery mechanism 100 is configured to transfer from the dose setting state to a dose delivery state upon a user pushing the button 330 in the proximal direction 5. Figs. 31A and 31B depicts the medicament delivery mechanism 100 in the dose delivery state prior to delivering the maximum dose.

The button 330 has moved in the proximal direction 5 with respect to the dose setting sleeve 250 and the coupling element 370 until the button 330 axially engages with the coupling element 370 via the bearing 360 and the washers 361. Thereby, the clutch mechanism between the dose setting element 310 and the coupling element 370 is opened so that the dose setting sleeve 250 and the coupling element 370 are allowed to rotate with respect to the dose setting element 310 during dose delivery.

Proximal movement of the button 330 also moves the coupling member 270 in the proximal direction 5 with respect to the dose setting sleeve 250 since the button 330 is axially coupled to the coupling member 270. This opens the clutch mechanism 275 between the coupling member 270 and the coupling part 370 so that the dose setting sleeve 250 and the coupling element 370 are allowed to rotate with respect to the coupling member 270 and the nut 200.

Further proximal movement of the button 330 then pushes the dose setting sleeve 250 in the proximal direction 5 since the proximal force exerted on the button 330 is transferred to the dose setting sleeve 250 via the bearing 360 and the coupling element 370. Due to the threaded connection between the outer thread 257 of the dose setting element 250 and the inner thread 163 of the body rear part 162 of the housing 160, the dose setting sleeve 250 rotates back into the housing 160. Proximal movement and rotation of the dose setting sleeve 250 also causes proximal movement of the drive sleeve 230 via the threaded connection between the outer thread 231 of the drive sleeve 230 and the inner thread 254 of the dose setting sleeve 250. Since the drive sleeve 230 is prevented from rotating by the rotation lock 202 it is screwed in the distal direction with respect to the dose setting member 250 due to the threaded connection between the outer thread 231 of the drive sleeve 230 and the inner thread 254 of the dose setting sleeve 250.

The drive sleeve 230 is axially coupled to the nut 200 in the proximal direction 5 and configured to push the nut 200 in the proximal direction 5, as it is further detailed below. Since the nut 200 is rotationally decoupled from the dose setting element 250 when the clutch mechanism 275 between the coupling member 270 and the coupling element 370 is in its opened state, it does not receive a torque from the rotating dose setting element 250. Rotation of the dose setting element 250 with respect to the nut 200 is further facilitated by the feedback mechanism 280 that allows rotation between the connector 270 and the dose setting sleeve 250 only during dose delivery.

The latching mechanism 191 acts as a rotation break during dose delivery that prevents the nut 200 from rotating with respect to the piston rod 110. This locks the threaded connection between the nut 200 and the piston rod 110 so that the piston rod 110 is forced to move axially together with the nut 200 in the proximal direction 5. The piston rod 110 and the piston 120 connected to the proximal end of the piston rod 110 then move into the container holder 40 and the medicament container 500 and advance the plunger 510 in the proximal direction 5 to expel the medicament from the medicament compartment 503.

The medicament delivery mechanism 100 comprises a zero dose stop that stops axial movement of the piston rod 110 in the proximal direction 5 at the end of dose delivery. The zero-dose stop may act between the dose setting sleeve 250 and the housing 160. It limits proximal movement of the dose setting sleeve 250 with respect to the housing 160. It may be configured as an axial stop with stop elements that engage with each other by moving towards each other along the longitudinal axis 7. In this case, the stop elements comprise stop surfaces that are orientated generally perpendicular, such as perpendicular, to the longitudinal axis 7. According to another option, the zero-dose stop may be configured as a radial stop with stop elements that engage with each other by moving towards each other around the longitudinal axis 7.

According to another alternative, the zero-dose stop may be formed between the dose setting element 310 and the housing 160, e.g. the body rear part 162. A first stop element is thereby formed at the dose setting element 310 and a second stop element is formed at the housing 160. The first stop element is provided by the proximal end surface of the dose setting element 310 and the second stop element is provided by the distal end surface of the housing 160, e.g. at the body rear part 162, namely by the distal end surface of the body rear part 162. As can be seen from Fig. 29, the proximal end surface of the dose setting element 310 and the distal end surface of the housing 180 are spaced apart from each other in the zero-dose state, i.e. when no force in the proximal direction is applied to the button 330.

With alternative embodiments of the medicament delivery mechanism 1, the pitch of the threaded connection between the outer thread 112 of the piston rod 110 and the inner thread 201 of the nut 200 may also be larger than the pitch of the threaded connection between the housing 160 and the dose setting sleeve 250. Alternatively, the pitch of the threaded connection between the outer thread 112 of the piston rod 110 and the inner thread 201 of the nut 200 may also equal the pitch of the threaded connection between the housing 160 and the dose setting sleeve 250. With such an embodiment, the drive sleeve 230 may be connected to the dose setting sleeve 250 by an axial lock that axially fixes the drive sleeve 230 to the dose setting sleeve 250 while allowing relative rotation of the drive sleeve 230 with respect to the dose setting sleeve 250.

The present disclosure is, inter alia, directed at the following enumerated embodiments:
1. Medicament delivery mechanism (100) for dispensing a medicament (501) from a medicament container (500), the medicament delivery mechanism (100) comprising:
   a first member, for example a drive sleeve (230'),
   a second member, for example a dose setting sleeve (250'),
   wherein the first member (230') and the second member (250') are configured to rotate relative to each other during dose setting,
   and for example thereby axially move relative to each other during dose setting,
   wherein the first member (230') comprises at least one first maximum dose stop surface (236') of a maximum dose stop (290'),
   wherein the second member (250') comprises at least one second maximum dose stop surface (256') of the maximum dose stop (290'),
   wherein the first and second maximum dose stop surfaces (236', 256') are configured to come in contact with each other when the medicament delivery mechanism (100) is set to a maximum settable dose, and
   wherein the maximum dose stop (290') is configured as a radial dose stop.
2. Medicament delivery mechanism (100) according to embodiment 1,
   wherein the at least one radial first maximum dose stop surface (236') of the first member (230') is formed at an elastically bendable portion (238') of the first member (230') that is bendable in a radial direction.
3. Medicament delivery mechanism (100) according to embodiment 2,
   wherein the elastically bendable portion (238') has a width (238w') along the longitudinal axis (7) and a length (238I') along a circumferential direction (6) and wherein the length (238l') of the elastically bendable portion (238') is greater than the width (238w') of the elastically bendable portion (238').
4. Medicament delivery mechanism (100) according to embodiment 2 or 3,
   wherein the elastically bendable portion (238') has a free end with a front surface forming a radial front surface (238a').
5. Medicament delivery mechanism (100) according to embodiment 4,
   wherein the radial front surface (238a') at the free end is configured as the first maximum dose stop surface (236').
6. Medicament delivery mechanism (100) according to one of the abovementioned embodiments,
   wherein at least one of the first maximum dose stop surface (236') and the second maximum dose stop surface (256') form oblique surfaces to force the first member (230') towards the second member (250') when the first maximum dose stop surface (236') and the second maximum dose stop surface (256') come in contact with each other.
7. Medicament delivery mechanism (100) according to one of the abovementioned embodiments,
   wherein the first maximum dose stop surface (236') and/or the second maximum dose stop surface (256') is positioned in a plane (P) that is spaced apart from the centerline of the medicament delivery mechanism (100).
8. Medicament delivery mechanism (100) according to one of the abovementioned embodiments,
   wherein the first maximum dose stop surface (236') and the second maximum dose stop surface (256') are at least in sections formed to interlock with each other and/or to displace a part of one of the first member (230') and the second member (250') in the direction towards the other member of the first member (230') and the second member (250').
9. Medicament delivery mechanism (100) according to one of the abovementioned embodiments,
   wherein the medicament delivery mechanism (100) comprises a plurality of radial maximum dose stops (290').
10. Medicament delivery mechanism (100) according to embodiment 9,
   wherein the plurality of radial maximum dose stops (290') are distributed equally around the circumference of the first member (230') and/or the second member (250').
11. Medicament delivery mechanism (100) according to one of the abovementioned embodiments,
   wherein the first member (230') is at least partly arranged inside the second member (250').
12. Medicament delivery mechanism (100) according to one of the abovementioned embodiments,
   wherein the first member (230') extends through a generally cylindrical portion of the second member (250').
13. Medicament delivery mechanism (100) according to one of the abovementioned embodiments,
   wherein the first member (230') is linearly guided and rotationally fixed relative to a housing (160) of the medicament delivery mechanism (100) and/or
   wherein the second member (250') is threadedly coupled to a housing (160) of the medicament delivery mechanism (100).
14. Medicament delivery mechanism (100) according to one of the abovementioned embodiments,
   wherein the first member (230') is configured to transfer an axial force via a nut (200) to a piston rod (110) during dose delivery.
15. Medicament delivery mechanism (100) according to one of the abovementioned embodiments,
   wherein the first member (230') is configured to transfer an axial force from the second member (250') to the nut (200) during dose delivery,
   for example, wherein the first member (230') engages with and/or abuts the second member (250') and/or the nut (200) to transfer the axial force from the second member (250') to the nut (200) during dose delivery.
16. Medicament delivery mechanism (100) according to one of the abovementioned embodiments,
   wherein the first member (230') forms a first thread (231') and the second member (250') forms a second thread (254') engaging the first thread (231').
17. Medicament delivery mechanism (100) according to embodiment 16,
   wherein the first maximum dose stop surface (236') is spaced apart from a possible end surface of the first thread (231') and/or the second maximum dose stop surface (256') is spaced apart from a possible end surface of the second thread (254').
18. Medicament delivery mechanism (100) according to embodiment 16 or 17,
   wherein the first maximum dose stop surface (236') does not form a delimiting surface of the first thread (231') and/or the second maximum dose stop surface (256') does not form a delimiting surface of the second thread (254').
19. Medicament delivery mechanism (100) according to embodiment 16, 17 or 18, wherein the first maximum dose stop surface (236') is configured separate from the first thread (231') and/or the second maximum dose stop surface (256') is configured separate from the second thread (254').
20. Medicament delivery mechanism (100) according to one of embodiments 16 to 19,
   wherein the first thread (231') is a single-start thread and the second thread (254') is a single-start thread.
21. Medicament delivery mechanism (100) according to one of the embodiments 16 to 20,
   wherein a number of the radial maximum dose stops (290') is larger than a number of starts of the first thread (231') and
   a number of starts of the second thread (254').
22. Medicament delivery mechanism (100) according to one of the abovementioned embodiments,
   wherein the first member (230') is a single integral part.
23. Medicament delivery mechanism (100) according to one of the abovementioned embodiments,
   wherein the second member (250') is a single integral part.
24. Medicament delivery mechanism (100) according to one of the abovementioned embodiments,
   wherein the at least one first radial maximum dose stop surface (236') is arranged at a distal end portion of the first member (230').
25. Medicament delivery mechanism (100) according to one of the abovementioned embodiments,
   wherein the medicament delivery mechanism (100) comprises a housing (160), in particular
   wherein the housing (160) comprises a body front part (180) and a body rear part (162)
   wherein the body front part (180) and the body rear part (162) form separate members that are fixedly connected to each other.
26. Medicament delivery mechanism (100) according to embodiment 25,
   wherein the housing (160), in particular the body front part (180), forms a linear guide (193) for the first member (230') that rotationally fixes the first member (230') to the housing (160) and/or
   wherein the housing (160), in particular the body rear part (162), forms a thread (163) that meshes with a thread (257') of the second member (250').
27. Medicament delivery mechanism (100) according to embodiment 25 or 26,
   wherein the medicament delivery mechanism (100) comprises a piston rod (110) extending from the housing (160), in particular the body front part (180), for advancing a plunger (510) and/or a piston (120) within the medicament container (500) to deliver the medicament (501).
28. Medicament delivery mechanism (100) according to embodiment 27,
   wherein the medicament delivery mechanism (100) comprises a nut (200), wherein the nut (200) forms a threaded connection (201, 112) with the piston rod (110).
29. Medicament delivery mechanism (100) according embodiment 28,
   wherein the housing (160), in particular the body front part (180), forms a linear guide (193) for the nut (200).
30. Medicament delivery mechanism (100) according embodiment 28 or 29,
   wherein the nut (200) comprises at least one flexible arm (222) configured to interact with a toothed inner circumferential surface (192) of the housing (160), in particular the body front part (180), by snapping into one of the spaces between two teeth of the toothed inner circumferential surface (192) to hold the nut (200) in a rotational position relative to the housing (160), in particular the body front part (180).
31. Medicament delivery mechanism (100) according embodiment 29 and 30,
   wherein the toothed inner circumferential surface (192) forms the linear guide (193) for the nut (200).
32. Medicament delivery mechanism (100) according to one of embodiments 25 to 31,
   wherein the housing (160), in particular the body front part (180), forms a container connector (90) to connect the medicament container (500) to the housing (160), in particular the body front part (180).
33. Medicament delivery mechanism (100) according to embodiment 32,
   wherein the container connector (90) is a snap fit connector, such as a non-releasable snap fit connector.
34. Medicament delivery mechanism (100) according to one of embodiments 25 to 33,
   wherein the body front part (180) and the body rear part (162) form corresponding and mutual engaging body connectors (183, 165) to connect the body front part (180) and the body rear part (162).
35. Medicament delivery mechanism (100) according to embodiment 34,
   wherein the body connectors (183, 165) are configured as snap fit connectors.
36. Medicament delivery mechanism (100) according to one of embodiments 25 to 35,
   wherein the body front part (180) is at least partly disposed within the body rear part (162).
37. Medicament delivery mechanism (100) according to one of embodiments 25 to 36,
   wherein the body rear part (162) forms an outer housing defining an outer surface (166) of the medicament delivery mechanism (100).
38. Medicament delivery mechanism (100) according to one of embodiments 25 to 37,
   wherein the body front part (180) forms an outer surface (181) of the medicament delivery mechanism (100), the outer surface (181) being, for example, located proximally from the outer surface (166) formed by the body rear part (162).
39. Medicament delivery mechanism (100) according to embodiment 26,
   wherein the linear guide (193) for the first member (230') formed by the housing (160), in particular by the body front part (180), comprises a toothed inner circumferential surface (192).
40. Medicament delivery mechanism (100) according to one of embodiments 25 to 39,
   wherein the housing (160), in particular the body front part (180), forms a linear guide (194) for the piston rod (110) that rotationally fixes the piston rod (110) to the housing (160), in particular the body front part (180).
41. Medicament delivery mechanism (100) according to one of embodiments 25 to 40,
   wherein the housing (160), in particular the body rear part (162), forms a threaded connection (231, 163) with the second member (250'), in particular the dose setting sleeve (250').
42. Medicament delivery mechanism (100) according to one of embodiments 28 to 41,
   wherein the medicament delivery mechanism (100) comprises a clutch mechanism (275),
   wherein the clutch mechanism (275) rotationally fixes the nut (200) to the second member (250'), in particular the dose setting sleeve (250'), during dose setting and rotationally decouples the nut (200) from the second member (250'), in particular the dose setting sleeve (250'), during dose delivery.
43. Medicament delivery mechanism (100) according to one of the abovementioned embodiments,
   wherein the medicament delivery mechanism (100) comprises a dose setting element (310) for setting at least one dose, and
   wherein the second member (250') is rotationally fixed to the dose setting element (310) during dose setting.
44. Medicament delivery mechanism (100) according to embodiment 42 and 43,
   wherein the clutch mechanism (275) comprises a coupling element (370) and a coupling member (270),
   wherein the coupling member (270) is rotatably fixed to the nut (200) during dose setting and dose delivery, and
   wherein the dose setting element (310) is rotationally fixed to the second member (250'), in particular the dose setting sleeve (250'), and the coupling member (270) via said coupling element (370) during dose setting.
45. Medicament delivery mechanism (100) according to any one of the preceding embodiments 25 to 44,
   wherein the second member (250'), in particular the dose setting sleeve (250'), comprises a marking (252) corresponding to the at least one dose on its outer circumferential surface and
   wherein the marking (252) is visible through an opening or a transparent window (164) in the housing (160), in particular the body rear part (162).
46. Medicament delivery mechanism (100) according to any one of the preceding embodiments 25 to 44,
   wherein the medicament delivery mechanism (100) comprises a button (330) forming a push surface (331) for delivery of the medicament, wherein axial movement of the button (330) relative to the housing (160), in particular the body rear part (162), during dose delivery causes the second member (250'), in particular the dose setting sleeve (250'), to rotate due to a threaded connection (257, 163) between the housing (160), in particular the body rear part (162), and the second member (250'), in particular the dose setting sleeve (250').
47. Medicament delivery device (1) comprising a medicament delivery mechanism (100)
   according to one of the preceding embodiments 25 to 46, and
   a medicament container (500) attached or attachable to the housing (160), in particular the body front part (162), wherein the medicament container (500) includes a medicament (501).
48. Medicament delivery device (1) according to embodiment 47,
   comprising a container holder (40), wherein the medicament container (500) is held within the container holder (40), for example by a snap fit connection.

### List of reference signs

- 1: medicament delivery device
- 2: proximal end
- 3: distal end
- 5: proximal direction
- 6: circumferential direction
- 7: longitudinal axis
- 10: cap
- 11: inner space
- 21: axial lock part
- 22: rotational lock part
- 32: protruding part
- 40: container holder
- 41: inner space
- 42: container connector
- 43: needle connector
- 44: axial cap lock part
- 45: rotational cap lock part
- 46: alignment part
- 47: axial coupling part
- 48: rotational coupling part
- 49: window
- 90: container connector
- 100: medicament delivery mechanism
- 110: piston rod
- 112: outer thread
- 113: connecting portion
- 114: last dose stop
- 120: piston
- 122: connector
- 152: biasing member
- 160: housing
- 162: body rear part
- 163: thread (dose setting sleeve)
- 164: window
- 165: body connector
- 166: outer surface
- 180: body front part
- 181: outer surface
- 182: coupling part
- 183: body connector
- 184: rotational coupling part
- 191: latching mechanism
- 192: toothed inner circumferential surface
- 192a: tooth/dose stop
- 192b: tooth/dose stop
- 192c: tooth/dose stop
- 193: linear guide (nut & drive sleeve)
- 194: linear guide (piston rod)
- 200: nut
- 201: inner thread (piston rod)
- 202: rotation lock
- 203: depression
- 205: body
- 206: outer surface
- 208: stop
- 209: first stop element
- 210: first contact structure
- 214: distal end face
- 219: second contact surface
- 220: protruding part
- 221: bar
- 222: flexible arm
- 223: latching part (longitudinal element)
- 224: rigid arm
- 230: drive sleeve
- 231: outer thread
- 232: body
- 236: first maximum dose stop surface
- 237: second stop element
- 238: elastically bendable portion
- 238a: radial front surface
- 238w: width
- 238I: length
- 240: protruding part
- 241: lock element
- 244: contact surface
- 250: dose setting sleeve
- 251: connector
- 252: marking
- 254: inner thread (to drive sleeve)
- 256: second maximum dose stop surface
- 257: outer thread (to housing)
- 258: toothed inner surface
- 270: connector/coupling member
- 271: clutch part
- 272: connection part
- 273: rotation lock
- 274: ridge
- 275: first clutch mechanism
- 276: first clutch part
- 277: distal cylindrical part
- 278: shoulder
- 279: flexible part
- 280: feedback mechanism
- 290: maximum dose stop
- 310: dose setting element
- 311: groove
- 312: gripping structure
- 313: second coupling section
- 330: button
- 331: push surface
- 332: projections
- 335: axial lock
- 360: bearing
- 361: washer
- 370: coupling element
- 371: second clutch part
- 372: first coupling section
- 373: contact surface
- 380: second clutch mechanism
- 500: medicament container
- 501: medicament
- 502: container body
- 503: medicament compartment
- 504: septum
- 505: connector
- 510: plunger
- P: plane

## Claims

1. Medicament delivery mechanism (100) for dispensing a medicament (501) from a medicament container (500), the medicament delivery mechanism (100) comprising:
a body front part (180),
a body rear part (162),
a dose setting element (310) for setting at least one dose,
a dose setting sleeve (250) rotationally fixed to the dose setting element (310) during dose setting,
a piston rod (110) extending from the body front part (180) for advancing a plunger within the medicament container (500) to deliver the medicament (501),
a nut (200), wherein the nut (200) forms a threaded connection (112, 201) with the piston rod (110),
a drive sleeve (230),
wherein the body front part (180) forms a container connector (90) to connect the medicament container (500),
wherein the body front part (180) is at least partly disposed within the body rear part (162),
wherein the body front part (180) forms a linear guide (193) for the drive sleeve (230) that rotationally fixes the drive sleeve (230) to the body front part (180),
wherein the body front part (180) and the nut (200) are rotatable to each other during dose setting,
wherein the body front part (180) comprises at least one dose stop (192a), wherein the dose stop (192a) is configured to hold the nut (200) in a predefined relative rotational position to the body front part (180), the relative rotational position corresponding to the at least one settable dose, and
wherein the body rear part (162) forms an outer housing defining an outer surface (166) of the medicament delivery mechanism (100).

2. Medicament delivery mechanism (100) according to claim 1,
wherein the body front part (180) and the body rear part (162) form separate members that are fixedly connected to each other,
and/or
wherein the dose setting element (310) is configured to set one of a plurality of settable doses, and wherein the body front part (180) comprises a plurality of dose stops (192a, 192b, 192c), wherein each of the plurality of dose stops (192a, 192b, 192c) is configured to hold the nut (200) in a predefined relative rotational position to the body front part (180), each relative rotational position corresponding to one of the plurality of settable doses.

3. Medicament delivery mechanism (100) according to claim 1 or 2,
wherein the at least one dose stop (192a) is located on a circumferential surface (192) of the body front part (180),
for example wherein the plurality of dose stops (192a, 192b, 192c) are evenly distributed along the circumferential surface (192) of the body front part (180).

4. Medicament delivery mechanism (100) according to at least one of the preceding claims,
wherein the at least one dose stop (192a) is located on an inner surface (192) of the body front part (180),
and/or
wherein the at least one dose stop (192a) is configured to directly engage with the nut (200) to hold the nut (200) in a predefined relative rotational position to the body front part (180).

5. Medicament delivery mechanism (100) according to at least one of the preceding claims,
wherein the at least one dose stop (192a) forms part of a latching mechanism (191) acting between the body front part (180) and the nut (200),
and/or
wherein the at least one dose stop (192a) is configured as a longitudinal ridge.

6. Medicament delivery mechanism (100) according to at least one of the preceding claims,
wherein the body front part (180) forms a linear guide (194) for the piston rod (110) that rotationally fixes the piston rod (110) to the body front part (180),
and/or
wherein the body rear part (162) forms a threaded connection (163, 257) with the dose setting sleeve (250).

7. Medicament delivery mechanism (100) according to at least one of the preceding claims,
wherein the container connector (90) is a snap-fit connector, such as a non-releasable snap-fit connector,
and/or
wherein the body front part (180) and the body rear part (162) form corresponding and mutual engaging body connectors (183, 165) to connect the body front part (180) and the body rear part (162), wherein, for example, the body connectors (183, 165) are configured as snap-fit connectors.

8. Medicament delivery mechanism (100) according to at least one of the preceding claims,
wherein the body front part (180) forms an outer surface (181) of the medicament delivery mechanism (100), the outer surface (181) being, for example, located proximally from the outer surface (166) formed by the rear body part (162),
and/or
wherein the body front part (180) forms a linear guide (193) for the nut (200).

9. Medicament delivery mechanism (100) according to at least one of the preceding claims,
wherein the linear guide (193) for the drive sleeve (230) formed by the body front part (180) comprises a toothed inner circumferential surface (192),
for example wherein at least one tooth (192a) of the toothed inner circumferential surface (192) forms the at least one dose stop (192a),
in particular wherein multiple teeth (192a, 192b, 192c) of the toothed inner circumferential surface (192) form a plurality of dose stops (192a, 192b, 192c).

10. Medicament delivery mechanism (100) according to claim 9,
wherein the nut (200) comprises at least one flexible arm (191a) configured to interact with the toothed inner circumferential surface (192) of the body front part (180) by snapping into one of the spaces between two teeth (192a, 192b) of the toothed inner circumferential surface (192) to hold the nut (200) in a rotational position relative to the body front part (180).

11. Medicament delivery mechanism (100) according claim 9 or 10,
wherein the toothed inner circumferential surface (192) forms a linear guide (193) for the nut (200).

12. Medicament delivery mechanism (100) according to any one of the preceding claims,
wherein the drive sleeve (230) forms an outer thread (231) meshing with an inner thread (254) of the dose setting sleeve (250),
and/or
further comprising a maximum dose stop (290),
wherein the maximum dose stop (290) is an axial or radial maximum dose stop (290),
wherein the maximum dose stop (290) acts between the dose setting sleeve (250) and the drive sleeve (230),
for example
wherein the drive sleeve (230) forms a surface (236) of the maximum dose stop (290) and/or wherein the surface (236) of the maximum dose stop (290) is configured to get in contact with the dose setting sleeve (250) when a maximum dose is set.

13. Medicament delivery mechanism (100) according to any one of the preceding claims,
wherein the medicament delivery mechanism (100) comprises a clutch mechanism (275),
wherein the clutch mechanism (275) rotationally fixes the nut (200) to the dose setting sleeve (250) during dose setting and rotationally decouples the nut (200) from the dose setting sleeve (250) during dose delivery,
for example wherein the clutch mechanism (275) comprises a coupling member (270) and a coupling element (370),
wherein the coupling member (270) is rotatably fixed to the nut (200) during dose setting and dose delivery, and
wherein the dose setting element (310) is rotationally fixed to the dose setting sleeve (250) and the coupling member (270) via said coupling element (25) during dose setting.

14. Medicament delivery mechanism (100) according to any one of the preceding claims,
wherein the dose setting sleeve (250) comprises a marking (252) corresponding to the at least one dose, for example on its outer circumferential surface and wherein the marking (252) is visible through an opening or a transparent window (164) in the body rear part (162),
and/or
wherein the medicament delivery mechanism comprises a button (330) forming a push surface (331) for delivery of the medicament (501), wherein axial movement of the button (330) relative to the body rear part (162) during dose delivery causes the dose setting sleeve (250) to rotate due to a threaded connection (257, 163) between the body rear part (162) and the dose setting sleeve (250).

15. Medicament delivery device (1) comprising a medicament delivery mechanism (100) according to one of the preceding claims, and
a medicament container (500) attached to the medicament delivery mechanism (100), e.g. to the body front part (180), wherein the medicament container (500) includes a medicament (501)
for example wherein the medicament delivery device (1) comprises a container holder (40), wherein the medicament container (500) is held within the container holder (40), for example by a snap fit connection.
